(19) Europäisches Patentamt
European Patent Office
Office européen des brevets

(11) EP 1 471 940 B1

(12) **EUROPEAN PATENT SPECIFICATION**

(45) Date of publication and mention of the grant of the patent:
**07.01.2009 Bulletin 2009/02**

(21) Application number: **03707711.2**

(22) Date of filing: **03.02.2003**

(51) Int Cl.:
***A61K 41/00*** (2006.01) ***A61K 31/7056*** (2006.01)

(86) International application number:
**PCT/US2003/003358**

(87) International publication number:
**WO 2003/063902 (07.08.2003 Gazette 2003/32)**

(54) **INACTIVATION OF WEST NILE VIRUS AND PLASMODIUM FALCIPARUM USING ALLOXAZINE-DERIVATING PHOTOSENSITISERS**

INAKTIVIERUNG DES WEST NILE VIRUS UND PLASMODIUM FALCIPARUM DURCH DIE VERWENDUNG VON ALLOXAZIN-DERIVATEN VON PHOTOSENSITIZERS

INACTIVATION DU VIRUS WEST NILE ET DE L'AGENT PATHOGENE DE LA MALARIA AU MOYEN DE PHOTOSENSIBILISANTS

(84) Designated Contracting States:
**DE FR GB IT**

(30) Priority: **01.02.2002 US 353162 P**

(43) Date of publication of application:
**03.11.2004 Bulletin 2004/45**

(73) Proprietor: **CaridianBCT Biotechnologies, LLC**
**Lakewood CO 80215 (US)**

(72) Inventors:
- **REDDY, Heather**
  **Denver, CO 80206 (US)**
- **GOODRICH, Raymond, P.**
  **Lakewood, CO 80227 (US)**

(74) Representative: **Cresswell, Thomas Anthony**
**J.A. KEMP & CO.**
**14 South Square**
**Gray's Inn**
**London**
**WC1R 5JJ (GB)**

(56) References cited:
**WO-A-00/04930**
**WO-A-01/28599**
**WO-A-01/78792**
**WO-A-01/96340**
**WO-A-94/28120**

- **SOLIMAN ET AL.: "Application of an immunoperoxidase monolyer assay for the detection of arboviral antibodies" JOURNAL OF VIROLOGICAL METHODS, vol. 65, no. 2, 1997, pages 147-151, XP002242775**
- **FRANCKI ET AL.: "Classification and nomenclature of viruses, Fifth report of the international committee on taxonomy of viruses, Archives of virology Supplementum 2" , SPRINGER-VERLAG , WIEN NEW-YORK XP002242777 page 223 -page 226**
- **APPLEYARD: "Photosensitivity of Semliki forest and other viruses" JOURNAL OF GENERAL VIROLOGY, vol. 1, - 1967 pages 143-152, XP008018664**
- **NJ WHITE ET AL: "The treatment of malaria" THE NEW ENGLAND JOURNAL OF MEDICINE, vol. 800, 1996, pages 800-806,**
- **"Random house webster's unabridged dictionary" WEBSTER * page 610 ***
- **HAWLEY: "Condensed chemical dictionary" RICHARD J LEWIS SR * pages 966,733, - pages 505,445 ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THE INVENTION

Contamination of blood supplies with infectious microorganisms such as malaria, West Nile virus, HIV, hepatitis and other viruses and bacteria presents a serious health hazard for those who must receive transfusions of whole blood or administration of various blood components such as platelets, red cells, blood plasma, Factor VIII, plasminogen, fibronectin, anti-thrombin III, cryoprecipitate, human plasma protein fraction, albumin, immune serum globulin, prothrombin complex, plasma growth hormones, and other components isolated from blood. Blood screening procedures may miss contaminants, and sterilization procedures which do not damage cellular blood components but effectively inactivate all infectious viruses and other microorganisms have not heretofore been available. In addition, a system that uses the same chemistry to inactivate microorganisms in different fluids, for example separate blood components, is desired for many reasons, including ease of use in a blood bank setting. This type of system has not heretofore been available. It is also desired that the inactivation treatment be easily implemented in a blood bank setting, and produce inactivation in a short period of time.

Malaria is an infectious disease caused by protozoan parasites of the *Plasmodium* genera. The species that cause malaria in humans are: *P. falciparum* (most malignant), *P. vivax, P. malariae*, and *P. ovale* (Mims, CA, et al. Medical Microbiology 1993; London: Mosby: 30.8-30.9). *Plasmodium* parasites are spread by the female anopheles mosquite, which transmits the infection to various primates and to non-immune human hosts (Boyd RF. Clinical parasitology. In Basic Medical Microbiology, 1995; Boston: Little, Brown and Company :513-514). Donated blood is not tested for infection with malaria, although there are no means to completely prevent the transmission of malaria by blood transfusion (Shulman I. Transmission of parasitic infections by blood transfusion. In Principles of transfusion medicine, eds. EC. Rossi, TL. Simon, GL. Moss, SA. Gould, 1996; Baltimore: Williams and Wilkins :733-8). In the United States, the risk of transfusion-transmitted malaria is limited by excluding blood donors who have traveled to malaria-endemic areas. This results in the deferral of 70,000 donors a year (Nahlen BL, et al. Reassessment of blood donor selection criteria for United States travelers to malarious areas. Transfusion. 1991;31:798-804). In countries with a high prevalence of malaria infection, deferral of donors may not be an option. A method for the inactivation of malaria parasites in blood may mitigate the risk of transfusion transmission from donors that are not removed through ordinary screening methods, and may allow the military to use donors that had been stationed in malaria-endemic areas. Such a method may also reduce the risk of transfusion-transmitted malaria in countries where large portions of the donor population have been exposed to the parasite.

There are several reported methods of decontaminating blood. Solvent detergent methods of blood component decontamination work by dissolving phospholipid membranes surrounding viruses such as HIV, and do not damage protein components of blood; however, if blood cells are present, such methods cannot be used because of damage to cell membranes.

The use of photosensitizers, compounds which absorb light of a defined wavelength and transfer the absorbed energy to an energy acceptor, has been proposed for blood component sterilization. For example, European Patent application 196,515 published October 8, 1986, suggests the use of non-endogenous photosensitizers such as porphyrins, psoralens, acridine, toluidines, flavine (acriflavine hydrochloride), phenothiazine derivatives, and dyes such as neutral red and methylene blue, as blood additives. Protoporphyrin, which occurs naturally within the body, can be metabolized to form a photosensitizer; however, its usefulness is limited in that it degrades desired biological activities of proteins. Chlorpromazine is also exemplified as one such photosensitizer; however its usefulness is limited by the fact that it should be removed from any fluid administered to a patient after the decontamination procedure because it has a sedative effect.

Goodrich, R.P., et al. (1997), "The Design and Development of Selective, Photoactivated Drugs for Sterilization of Blood Products," Drugs of the Future 22:159-171 provides a review of some photosensitizers including psoralens, and some of the issues of importance in choosing photosensitizers for decontamination of blood products. The use of texaphyrins for DNA photocleavage is described in U.S. Patent Nos. 5,607,924 issued March 4, 1997 and 5,714,328 issued February 3, 1998 to Magda et al. The use of sapphyrins for viral deactivation is described in U.S. Patent No. 5,041,078 issued August 20, 1991 to Matthews, et al. Inactivation of extracellular enveloped viruses in blood and blood components by Phenthiazin-5-ium dyes plus light is described in U.S. Patent No. 5,545,516 issued August 13, 1996 to Wagner. The use of porphyrins, hematoporphyrins, and merocyanine dyes as photosensitizing agents for eradicating infectious contaminants such as viruses and protozoa from body tissues such as body fluids is disclosed in U.S. Patent 4,915,683 issued April 10, 1990 and related U.S. Patent No. 5,304,113 issued April 19, 1994 to Sieber et al. The mechanism of action of such photosensitizers is described as involving preferential binding to domains in lipid bilayers, e.g. on enveloped viruses and some virus-infected cells. Photoexcitation of membrane-bound agent molecules leads to the formation of reactive oxygen species such as singlet oxygen which causes lipid peroxidation. A problem with the use of such photosensitizers is that they attack cell membranes of desirable components of fluids to be decontaminated,

such as red blood cells, and the singlet oxygen also attacks desired protein components of fluids being treated. U.S. Patent 4,727,027 issued February 23, 1988 to Wiesehahn, G.P., et al. discloses the use of furocoumarins including psoralen and derivatives for decontamination of blood and blood products, but teaches that steps must be taken to reduce the availability of dissolved oxygen and other reactive species in order to inhibit denaturation of biologically active proteins.

Photoinactivation of viral and bacterial blood contaminants using halogenated coumarins is described in U.S. Patent 5,516,629 issued May 14, 1996 to Park, et al. U.S. Patent 5,587,490 issued December 24, 1996 to Goodrich Jr., R.P., et al. and U.S. Patent No. 5,418,130 to Platz, et al. disclose the use of substituted psoralens for inactivation of viral and bacterial blood contaminants. The latter patent also teaches the necessity of controlling free radical damage to other blood components. U.S. Patent 5,654,443 issued August 5, 1997 to Wollowitz et al. teaches new psoralen compositions used for photodecontamination of blood. U.S. Patent 5,709,991 issued January 20, 1998 to Lin et al. teaches the use of psoralen for photodecontamination of platelet preparations and removal of psoralen afterward. U.S. Patent 5,120,649 issued June 9, 1992 and related U.S. Patent 5,232,844 issued August 3, 1993 to Horowitz, et al., also disclose the need for the use of "quenchers" in combination with photosensitizers which attack lipid membranes, and U.S. Patent 5,360,734 issued November 1, 1994 to Chapman et al. also addresses the problem of prevention of damage to other blood components.

Photosensitizers which attack nucleic acids are known to the art. U.S. Patent 5,342,752 issued August 30, 1994 to Platz et al. discloses the use of compounds based on acridine dyes to reduce parasitic contamination in blood matter comprising red blood cells, platelets, and blood plasma protein fractions. These materials, although of fairly low toxicity, do have some toxicity e.g. to red blood cells. This patent fails to disclose an apparatus for decontaminating blood on a flow-through basis. U.S. Patent No. 5,798,238 to Goodrich, Jr., et al., discloses the use of quinolone and quinolone compounds for inactivation of viral and bacterial contaminants.

Binding of DNA with photoactive agents has been exploited in processes to reduce lymphocytic populations in blood as taught in U.S. Patent No. 4,612,007 issued September 16, 1986 and related U.S. Patent No. 4,683,889 issued August 4, 1987 to Edelson.

Riboflavin (7,8-dimethyl-10-ribityl isoalloxazine) has been reported to attack nucleic acids. Photoalteration of nucleic acid in the presence of riboflavin is discussed in Tsugita, A, et al. (1965), Photosensitized inactivation of ribonucleic acids in the presence of riboflavin, Biochimica et Biophysica Acta 103:360-363; and Speck, W.T. et al. (1976), Further Observations on the Photooxidation of DNA in the Presence of Riboflavin, Biochimica et Biophysica Acta 435:39-44. Binding of lumiflavin (7,8,10-trimethylisoalloxazine) to DNA is discussed in Kuratomi, K., et al. (1977), Studies on the Interactions between DNA and Flavins, Biochimica et Biophysica Acta 476:207-217. Hoffmann, M.E., et al. (1979), DNA Strand Breaks in Mammalian Cells Exposed to Light in the Presence of Riboflavin and Tryptophan, Photochemistry and Photobiology 29:299-303 describes the use of riboflavin and tryptophan to induce breaks in DNA of mammalian cells after exposure to visible fluorescent light or near-ultraviolet light. The article states that these effects did not occur if either riboflavin or tryptophan was omitted from the medium. DNA strand breaks upon exposure to proflavine and light are reported in Piette, J. et al. (1979), Production of Breaks in Single- and Double-Stranded Forms of Bacteriophage ΦX174 DNA by Proflavine and Light Treatment, Photochemistry and Photobiology 30:369-378, and alteration of guanine residues during proflavine-mediated photosensitization of DNA is discussed in Piette, J., et al. (1981), Alteration of Guanine Residues during Proflavine Mediated Photosensitization of DNA, Photochemistry and Photobiology 33:325-333.

J. Cadet, et al. (1983), Mechanisms and Products of Photosensitized Degradation of Nucleic Acids and Related Model Compounds, Israel J. Chem. 23:420-429, discusses the mechanism of action by production of singlet oxygen of rose bengal, methylene blue, thionine and other dyes, compared with mechanisms not involving production of singlet oxygen by which nucleic acid attack by flavin or pteron derivatives proceeds. Riboflavin is exemplified in this disclosure as having the ability to degrade nucleic acids. Korycka-Dahl, M., et al. (1980), Photodegradation of DNA with Fluorescent Light in the Presence of Riboflavin, and Photoprotection by Flavin Triplet-State Quenchers, Biochimica et Biophysica Acta 610:229-234 also discloses that active oxygen species are not directly involved in DNA scission by riboflavin. Peak, J.G., et al. (1984), DNA Breakage Caused by 334-nm Ultraviolet Light is Enhanced by Naturally Occurring Nucleic Acid Components and Nucleotide Coenzymes, Photochemistry and Photobiology 39:713-716 further explores the mechanism of action of riboflavin and other photosensitizers. However, no suggestion is made that such photosensitizers be used for decontamination of medical fluids.

Addition of riboflavin to *in vitro* cultures of *P. falciparum* has been reported to inhibit asexual parasite growth (Akompong, T., et al. In Vitro Activity of Riboflavin against the Human Malaria Parasite Plasmodium falciparum., Antimicrob Agents Chemother, January 2000;44: 88-96) and kill gametocytes (Akompong, T., et al. Gametocytocidal Activity and Synergistic Interactions of Riboflavin with Standard Antimalarial Drugs against Growth of Plasmodium falciparum In Vitro. Antimicrob Agents Chemother, November 2000; 44: 3107-3111). Riboflavin, when added to cultures in the asexual stage in combination with antimalarial drugs, was reported to enhance the drug activity (Akompong, T., et al. Gametocytocidal Activity and Synergistic Interactions of Riboflavin with Standard Antimalarial Drugs against Growth of Plasmodium falciparum In Vitro. Antimicrob Agents Chemother, November 2000; 44: 3107-3111). In earlier works (Das

BS, et al. Riboflavin deficiency and severity of malaria, Eur J Clin Nutr, 1988 Apr;42:277-83; Dutta P. Enhanced uptake and metabolism of riboflavin in erythrocytes infected with Plasmodium falciparum. J Protozool 1991 Sep-Oct;38:479-83), riboflavin deficiency was observed to be detrimental to the parasite. The use of riboflavin as a photosensitizer to treat blood and blood components that may have malaria infection has not been reported.

Apparatuses for decontamination of blood have been described in U.S. Patent No. 5,290,221 issued March 1, 1994 to Wolfe, Jr., et al. and U.S. Patent No. 5,536,238 issued July 16, 1996 to Bischof. U.S. Patent No. 5,290,221 discloses the irradiation of fluid in a relatively narrow, arcuate gap. U.S. Patent 5,536,238 discloses devices utilizing optical fibers extending into a filtration medium. Both patents recommend as photosensitizers benzoporphryin derivatives which have an affinity for cell walls.

U.S. Patent No. 5,527,704 issued June 18, 1996 to Wolf, Jr., et al. discusses an apparatus to inactivate viruses contained in a body fluid in a container using methylene blue as a photosensitizer. The body fluid is maintained in a static state within the container during irradiation. U.S. Patent No. 5,868,695 issued February 9, 1999 to Wolf, Jr. et al. discloses a system where blood containing a photoactive material is directed in a predetermined flow path such as a serpentine in a narrow gap in a treatment chamber. PCT published application No. WO 96/06647 discloses irradiating a product in an array of light emitting diodes surrounded by a fluid used to prevent overheating of the diodes. Riboflavin and UV light inactivate viruses and bacteria in plasma and platelet products (Samar, R, *et al*. Poster, Viral Inactivation in Plasma Using Riboflavin-Based Technology. AABB 54th Annual Meeting. November 2001; Goodrich RP. The use of riboflavin for the inactivation of pathogens in blood products. *Vox Sang*. 2000;78 (suppl 2):211-15). Riboflavin and visible light provide demonstrated virus inactivation in platelets (Goodrich, L, *et al*. Poster. Riboflavin Pathogen Inactivation Process Yields Good Platelet Cell Quality and Expedient Viral Kill. ASH 43rd Annual Meeting. December 2001) and in red cell suspensions (McAteer MJ, *et al*. Poster: Photo-inactivation of virus in packed red blood cell units using riboflavin and visible light. AABB 53rd Annual Meeting. November 2000).

Sterilization procedures which do not damage cellular blood components but effectively inactivate infectious viruses and other microorganisms and contaminants are disclosed in U.S. Patents 6,258,577, 6,277,337, 6,268,120 and PCT publications WO 01/28599, WO 00/04930. Storage solutions containing photosensitizers are disclosed in U.S. Patent Application 09/725,426 and U.S. Patent Application 09/596,429.

There is a need for an inactivation procedure for West Nile virus and malaria, as well as other microorganisms that are not detected in blood products.

WO 00/04930, WO 01/28599 and WO 01/96340 discuss methods and apparatuses for treating fluids to inactivate microorganisms which may be present therein. WO 01/78792 provides a method for reducing the level of active pathogenic contaminants found in whole blood and blood components. WO 94/28120 concerns the product produced by inactivating extracellular or intracellular pathogenic virus in a biological composition.

G. Appleyard (J. Gen. Virol. (1967), 1, 143-152) discusses inactivation of various viruses, in particular Semliki Forest virus.

R Schuyler (Transfusion and Apheresis Science 25, (2001), 189-190) discusses the use of riboflavin as a photosensitizer having an extremely good safety profile. It suggests that riboflavin may inactivate high levels of a broad range of viruses and bacteria in platelet concentrations, fresh frozen plasma, and in red blood cells, preserving the activity and functionality of the components. No mention, however, is made of the inactivation of viruses.

## SUMMARY OF THE INVENTION

The present invention provides a method for treating a fluid to inactivate at least the parasite *P. falciparum* that may be present therein, said fluid containing one or more components selected from human whole blood, human blood constituents, or aqueous compositions containing biologically active protein derived from human blood, the method comprising:

(a) adding an inactivation effective, substantially non-toxic amount of a photosensitizer which is an endogenous alloxazine or endogenously-based derivative alloxazine to the fluid; and
(b) exposing the fluid of step (a) to photoradiation sufficient to activate the photosensitizer, whereby at least any *P. falciparum* present in the fluid is inactivated.

Preferably, said fluid of step (a) is exposed to a source of photoradiation at a depth selected to ensure penetration of the photoradiation through the fluid and inactivation of the parasite.

Preferably, the photosensitiser is an endogenous alloxazine.

The means for maintaining the fluid and an effective amount of an endogenous or endogenously-based derivative photosensitizer in the light path may comprise a support surface substantially parallel to said source of light; a cuvette or bag; or other means known in the art.

The photopermeable container may be a transparent plastic bag, a transparent plastic container with rigid walls,

or other containers as known to the art. The agitation may be provided by a shaker table, or other means for agitating known to the art.

The photosensitizer may be a photo-activatable compound whose photolytic products (if any) are of low or no toxicity to humans or animals. The most preferred photosensitizer is 7,8-dimethyl-10-ribityl isoalloxazine. The photosensitizer is preferably a nucleic-acid-targeted non toxic photoactivatable compound which does not produce toxic photolytic breakdown products. Preferred photosensitizers are not porphyrin.

Photoradiation may comprise light in the visible spectrum, the ultraviolet spectrum, or light in both the visible and ultraviolet spectra. Any suitable wavelength or wavelengths of light may be used in any proportion and energy that produces the desired level of inactivation of microorganisms. As used herein, wavelength does not necessarily mean one discrete wavelength. Wavelength may comprise a range of about ± 100 nm centered around one wavelength. Preferably, if ultraviolet light is used, the amount of ultraviolet light is kept to a level that minimizes damage to desired fluid components. Generally, this is provided by using 50% or less ultraviolet light relative to the total light energy delivered. Also, the wavelengths of light may be centered around one peak wavelength (i.e., a range of ± 10 nm centered around one wavelength).

Preferably, photoirradiation comprises one or more wavelengths in the visible spectrum.

Preferably, photoirradiation comprises one or more wavelengths in the ultraviolet spectrum.

The fluid containing the photosensitizer is exposed to photoradiation of the appropriate wavelength to activate the photosensitizer, using an amount of photoradiation sufficient to activate the photosensitizer as described herein, but less than that which would cause non-specific damage to the biological components or substantially interfere with biological activity of other proteins present in the fluid. The wavelength used will depend on the photosensitizer selected and composition of the fluid, as is known to the art or readily determinable without undue experimentation following the teachings disclosed herein. Non-specific damage is damage that damages all components.

The photoradiation in both the ultraviolet and visible spectra may be supplied concurrently or sequentially, with the visible portion preferably being supplied first. The photoradiation source may be a simple lamp or may consist of multiple lamps radiating at differing wavelengths. The photoradiation source should be capable of delivering a sufficient amount of light to activate the photosensitizer, preferably from about 1 to at least about 200 $J/cm^2$, most preferably around 7 $J/cm^2$. All values and ranges of power are included herein.

As used herein, the term inactivation of a microorganism means totally or partially preventing the microorganism from replicating, either by killing the microorganism or otherwise interfering with its ability to reproduce.

Microorganisms include viruses (both extracellular and intracellular), bacteria, bacteriophages, fungi, blood-transmitted parasites such as malaria, and protozoa. Exemplary viruses include acquired immunodeficiency (HIV) virus, hepatitis A, B and C viruses, sinbis virus, cytomegalovirus, vesicular stomatitis virus, herpes simplex viruses, e.g. types I and II, human T-lymphotropic retroviruses, HTLV-III, lymphadenopathy virus LAV/IDAV, parvovirus, transfusion-transmitted (TT) virus, Epstein-Barr virus, West Nile virus and others known to the art. Bacteriophages include ΦX174, Φ6, λ, R17, $T_4$, and $T_2$. Exemplary bacteria include *P. aeruginosa*, *S. aureus*, *S. epidermis*, *L. monocytogenes*, *E. coli*, *K. pneumonia* and *S. marcescens*. One particular class of microorganisms is non-screened microorganisms-those microorganisms that are not screened by current blood banking processes. Some non-screened microorganisms include malaria and West Nile virus. One class of microorganisms include those transmitted by mosquitoes, including malaria and West Nile virus.

Materials which may be treated by the methods of this invention include any materials which are adequately permeable to photoradiation to provide sufficient light to achieve microorganism inactivation, or which can be suspended or dissolved in fluids which have such permeability to photoradiation. Examples of such materials are whole blood and aqueous compositions containing biologically active proteins derived from blood or blood constituents. Packed red cells, platelets and plasma (fresh or fresh frozen plasma) are exemplary of such blood constituents. In addition, therapeutic protein compositions containing proteins derived from blood, such as fluids containing biologically active protein useful in the treatment of medical disorders, e.g. factor VIII, Von Willebrand factor, factor IX, factor X, factor XI, Hageman factor, prothrombin, anti-thrombin III, fibronectin, plasminogen, plasma protein fraction, immune serum globulin, modified immune globulin, albumin, plasma growth hormone, somatomedin, plasminogen streptokinase complex, ceruloplasmin, transferrin, haptoglobin, antitrypsin and prekallikrein may be treated by the decontamination methods of this invention. The activity of a biologically-active protein in said fluid is at a biologically-active level after said exposing step. A therapeutic protein present in said fluid remains able to perform a therapeutic function after the exposing step.

Preferably, the fluid comprises human blood constituents. Preferably, the fluid is human blood. Preferably, the fluid is a separated human blood product.

The term biologically active means capable of effecting a change in a living organism or component thereof. Biologically active with respect to biologically active protein as referred to herein does not refer to proteins which are part of the microorganisms being inactivated. Similarly, non-toxic with respect to the photosensitizers means low or no toxicity to humans and other mammals, and does not mean non-toxic to the microorganisms being inactivated. Substantial destruction of biological activity means at least as much destruction as is caused by porphyrin and porphyrin derivatives,

metabolites and precursors which are known to have a damaging effect on biologically active proteins and cells of humans and mammals. Similarly, substantially non-toxic means less toxic than porphyrin, porphyrin derivatives, metabolites and precursors that are known for blood sterilization.

The term blood product as used herein includes blood constituents and therapeutic protein compositions containing proteins derived from blood as defined above. Fluids containing biologically active proteins other than those derived from blood may also be treated by the methods of this invention.

Decontamination methods of this invention using endogenous alloxazine photosensitizers and endogenously-based alloxazine derivative photosensitizers do not substantially destroy the biological activity of fluid components other than microorganisms. As much biological activity of these components as possible is retained, although in certain instances, when the methods are optimized, some loss of biological activity, e.g., denaturization of protein components, must be balanced against effective decontamination of the fluid. So long as fluid components retain sufficient biological activity to be useful for their intended or natural purposes, their biological activities are not considered to be substantially destroyed.

Photosensitizers are known to be useful for inactivating microorganisms. A photosensitizer is defined as any compound which absorbs radiation of one or more defined wavelengths and subsequently utilizes the absorbed energy to carry out a chemical process. Examples of such photosensitizers include porphyrins, psoralens, dyes such as neutral red, methylene blue, acridine, toluidines, flavine (acriflavine hydrochloride) and phenothiazine derivatives, coumarins, quinolones, quinones, and anthroquinones. Photosensitizers of this invention are endogenous alloxazine or endogenously-based derivative alloxazine. These may include compounds which preferentially adsorb to nucleic acids, thus focusing their photodynamic effect upon microorganisms and viruses with little or no effect upon accompanying cells or proteins. Other photosensitizers are also useful in this invention, such as those using singlet oxygen-dependent mechanisms. Most preferred are endogenous photosensitizers. The term endogenous means naturally found in a human or mammalian body, either as a result of synthesis by the body or because of ingestion as an essential foodstuff (e.g. vitamins) or formation of metabolites and/or byproducts *in* vivo. Examples of such endogenous photosensitizers are alloxazines such as 7,8-dimethyl-10-ribityl isoalloxazine (riboflavin), 7,8,10-trimethylisoalloxazine (lumiflavin), 7,8-dimethylalloxazine (lumichrome), isoalloxazine-adenine dinucleotide (flavine adenine dinucleotide [FAD]), alloxazine mononucleotide (also known as flavine mononucleotide [FMN] and riboflavine-5-phosphate). The term alloxazine includes isoalloxazines. Endogenously-based derivative photosensitizers include synthetically derived analogs and homologs of endogenous photosensitizers which may have or lack lower (1-5) alkyl or halogen substituents of the photosensitizers from which they are derived, and which preserve the function and substantial non-toxicity thereof. When endogenous or endogenously-based derivative photosensitizers are used, particularly when such photosensitizers are not inherently toxic or do not yield toxic photoproducts after photoradiation, no removal or purification step is required after decontamination, and treated product can be directly returned to a patient's body or administered to a patient in need of its therapeutic effect.

Non-endogenous photosensitizers based on endogenous structures, such as those described in U.S. Patent 6,268,120 are also useful. These non-endogenous photosensitizers and endogenously-based derivative photosensitizers are referred to herein as endogenously-based derivative photosensitizers.

Preferred photosensitizers (also referred to herein as photoactivators) are endogenous alloxazines, specifically 7,8-dimethyl-10-ribityl isoalloxazine, (riboflavin) 7,8-dimethylalloxazine, 7,8,10-trimethylisoalloxazine, alloxazine mononucleotide, isoalloxazine-adenosine dinucleotide, and isoalloxazine derivatives and analogs as set forth in U.S. Patent No. 6,268,120 and U.S. Patent Application 09/777,727. Specifically, the terms endogenously-based derivative photosensitizers and isoalloxazine derivative photosensitizers are synonymous and mean compounds having the structure:

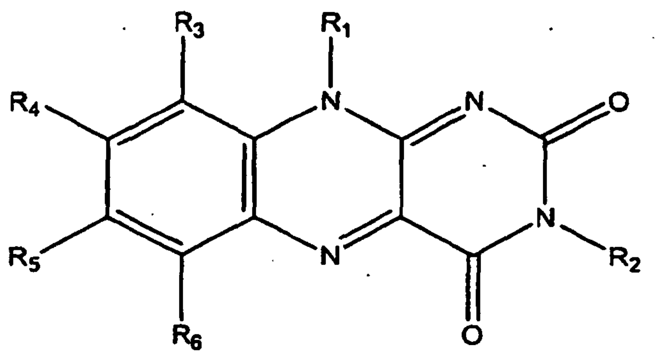

wherein $R_1$, $R_2$, $R_3$, $R_4$, $R_5$ and $R_6$ are, independently from one another, selected from the group consisting of hydrogen,

optionally substituted hydrocarbyl, alcohol, amine, polyamine, sulfate, phosphate, halogen selected from the group consisting of chlorine, bromine and iodine, salts of the foregoing, and $-NR^a-(CR^bR^c)_n-X$ wherein X is a halogen selected from the group consisting of chlorine, bromine and iodine, $R^a$, $R^b$ and $R^c$ are, independently of each other, selected from the group consisting of hydrogen, optionally substituted hydrocarbyl, and halogen selected from the group consisting of chlorine, bromine and iodine, and n is an integer from 0 to 20.

Preferred endogenously-based derivative photosensitizers are compounds having the structure:

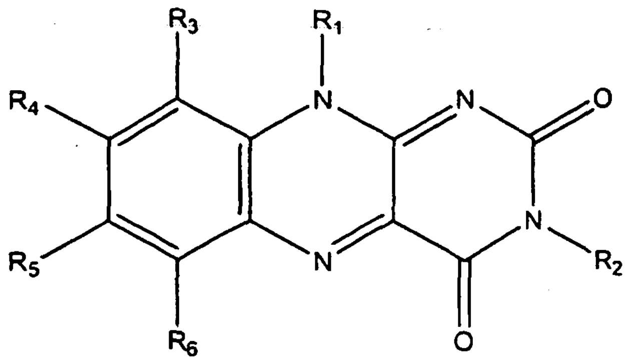

provided that $R_1$ is not -OH or a straight chain alkyl group where the second carbon of the chain is substituted with -OH or =O; and $R_1$, $R_4$, and $R_5$ are not all methyl groups when $R_2$, $R_3$, and $R_6$; are all hydrogen. In specifically exemplified classes of endogenously-based derivative photosensitizers, $R_1$ is not a 2-, 3-, 4- or 5- carbon straight chain alkyl that terminates in -OH, -COH, or -H when $R_2$, $R_3$ and $R_6$ are H, and $R_4$ and $R_5$ are $CH_3$; $R_1$ is not - $CH_2CH_2-(CHOH)_2-CH_3$ or $-CH_2CH_2-(CHOH)_2-CH_2SO_4$ or 1'-D-sorbityl or 1'-D-dulcityl or 1'-D-rhamnityl or 1'-D,L-glyceryl or $-CH_2-O-C(O)-CH_3$ or $-CH_2-O-C(O)-CH_2CH_3$ or 2', 3', 4', 5'-di-O-isopropyridene-riboflavin or 8-aminooctyl when $R_2$, $R_3$ and $R_6$ are H and $R_4$ and $R_5$ are $CH_3$; $R_1$ is not 1'-D-sorbityl or 1'-D-dulcityl when $R_4$ and $R_5$ are both chlorines and when $R_2$, $R_3$ and $R_6$ are all hydrogens; $R_5$ is not ethyl or chloro when $R_1$ and $R_4$ are methyl and $R_2$, $R_3$ and $R_6$ are all hydrogens; $R_4$ and $R_5$ are not both methoxy or both tetramethylene when $R_1$ is methyl and $R_2$, $R_3$ and $R_6$ are all hydrogens; $R_2$ is not $-CH_2CH_2NH$ when $R_1$, $R_4$ and $R_5$ are $CH_3$ and $R_3$ and $R_6$ are H; $R_2$ is not

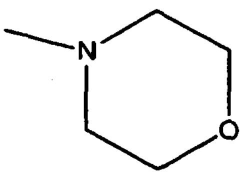

when $R_1$, $R_4$ and $R_5$ are $CH_3$ and $R_3$ and $R_6$ are H; $R_5$ is not chloro when $R_4$ is methoxy and $R_1$ is ethyl-2'N-pyrrolidino and $R_2$, $R_3$, and $R_6$ are hydrogen; $R_1$ is not N,N-dimethylaminopropyl or N,N-diethylaminoethyl when $R_5$ is chloro or methyl and $R_2$, $R_3$, $R_4$ and $R_6$ are hydrogen; $R_3$ is not $-NH(CH_2CH_2)Cl$ when $R_6$ is $-NH_2$ and $R_1$, $R_2$, $R_4$ and $R_5$ are H; $R_1$, $R_4$, $R_5$ are not all methyl groups when all of $R_2$, $R_3$ and $R_6$ are hydrogens; R1 and R2 are not both methyl groups when R3, R4, R5 and R6 are H; $R_1$, $R_4$, $R_5$ and $R_2$ are not all methyl groups when $R_3$ and $R_6$ are hydrogens; $R_2$ is not carboxymethyl when $R_1$, $R_4$ and $R_5$ are methyl and $R_3$ and $R_6$ are hydrogen; $R_4$ is not $-NH_2$ when $R_1$ and $R_5$ are methyl and $R_2$, $R_3$ and $R_6$ are all hydrogen; $R_1$ is not a phenyl group when $R_4$ and $R_5$ are methyl and $R_2$, $R_3$ and $R_6$ are all H; $R_1$ is not methyl or N,N-dimethylaminoethyl when all of $R_2$, $R_3$, $R_4$, $R_5$ and $R_6$ are hydrogen; $R_2$, $R_4$, $R_5$ are not all methyl when $R_1$ is acetoxyethyl and $R_3$ and $R_6$ are hydrogen; $R_5$ is not methyl when $R_1$ is N,N-diethylaminoethyl and $R_2$, $R_3$, $R_4$ and $R_6$ are all hydrogen; $R_4$ and $R_5$ are not both chlorine when $R_1$ is methyl and $R_2$, $R_3$ and $R_6$ are all hydrogen; $R_1$ is not ethyl, β-chloroethyl, nBbutyl, anilino, benzyl, phenyl, p-tolyl or p-anisyl when $R_5$ is $NH_2$ and $R_2$, $R_3$, $R_4$ and $R_6$ are all hydrogen; and $R_4$ is not chlorine when $R_1$ is N,N-dimethylaminopropyl and $R_2$, $R_3$, $R_5$ and $R_6$ are all hydrogen.

In one group of compounds, n is an integer between 0 and 5. In another group of compounds, n is an integer from 0 to 10. In another group of compounds, n is an integer from 0 to 20.

Compounds containing any combination of substituents or members of the Markush groups specified above may be used in the method of the invention. All such compounds have the ability to neutralize microorganisms. All substituents of the compounds may be the same, all substituents may be different, or any combination of substituents

may be the same or different. Substituents with a specified function, for example those that impart water solubility to the compound, may be included at any of $R_{1-6}$. Compounds suitable for use in the present invention include all those compounds with the isoalloxazine backbone (shown below):

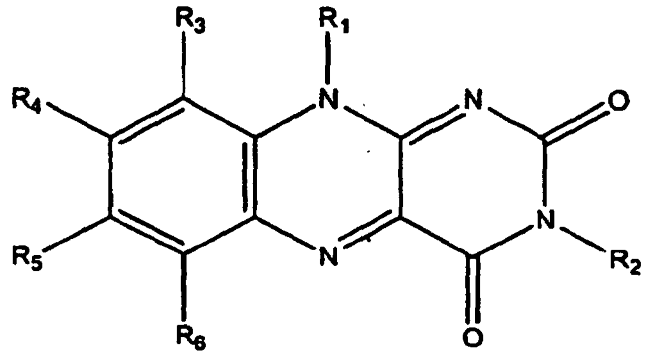

where $R_1$-$R_6$ are substituted with various substituents, as described elsewhere, except those previously known to the art. The substituents included in the compounds used in the methods of the invention may be any substituent not having structures or reactivity which would substantially interfere with the desired microorganism neutralization of the microorganism neutralizer, as may readily be determined without undue experimentation by those skilled in the art.

The invention may utilise a class of compounds wherein one or a plurality of $R_1$, $R_2$, $R_3$, $R_4$, $R_5$ and $R_6$ are neither $CH_3$ nor H; and a class of compounds wherein one of $R_1$, $R_2$, $R_3$, $R_4$, $R_5$ and $R_6$ is neither $CH_3$ nor H. The invention may also utilise a class of compounds wherein one or a plurality of R1-R6 are $CH_3$ or H. Particular embodiments of compounds of those classes include those wherein a $R_1$, $R_2$, $R_3$, $R_4$, $R_5$ or $R_6$ which is neither $CH_3$ nor H imparts substantial water solubility to the microorganism neutralizer. Preferred examples of these compounds are:

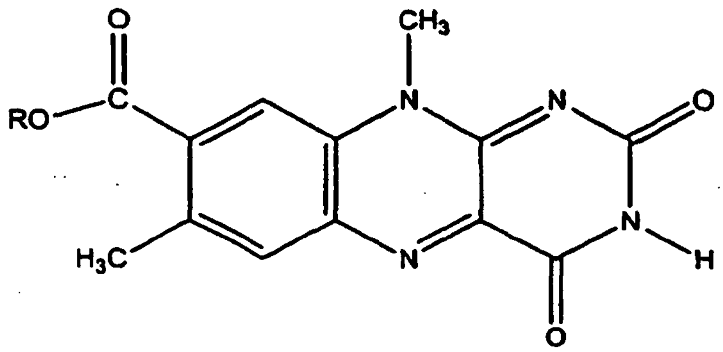

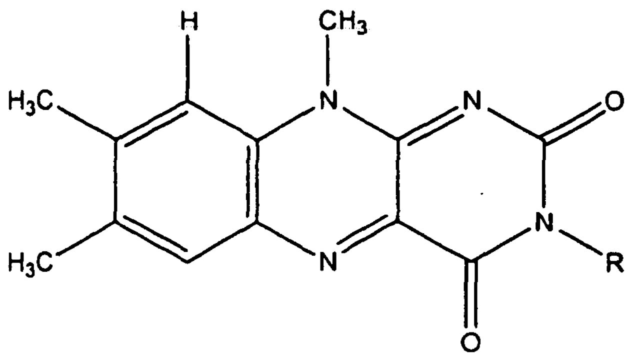

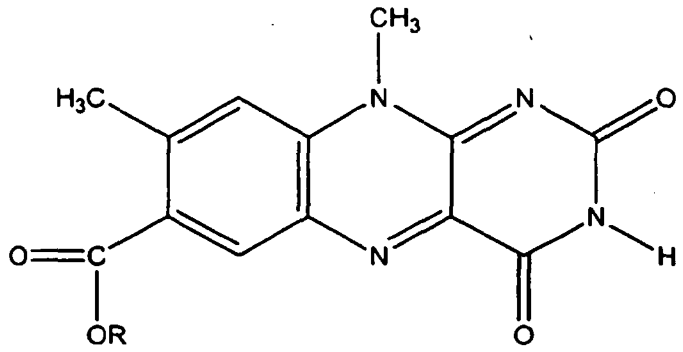

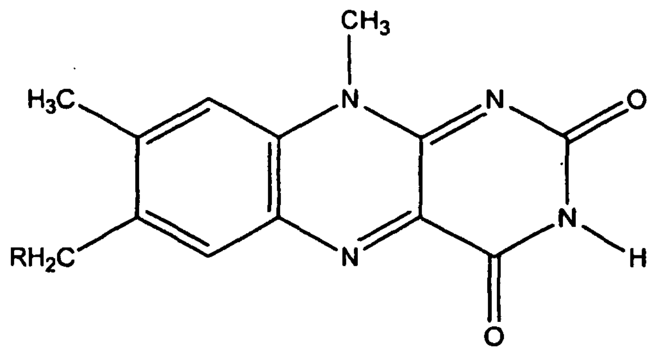

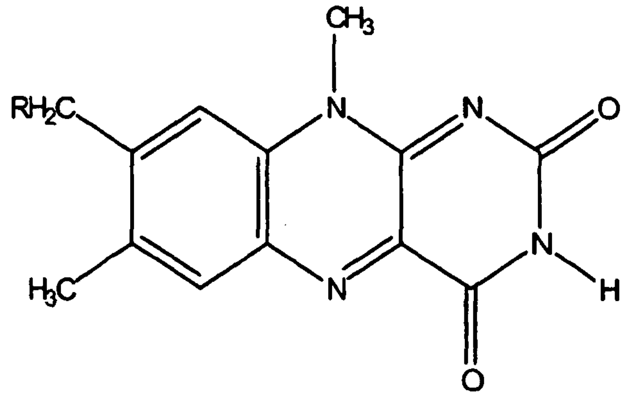

wherein R is a substituent imparting water solubility to the molecule, including, but not limited to, ascorbate, alcohol, polyalcohol; amine or polyamines, straight chain or cyclic saccharides, sulfates, phosphates, alkyl chains optionally substituted with -OH at any position, glycols, including polyethylene glycol and polyethers.

Another class of compounds suitable for use in the present invention include those wherein a $R_1$, $R_2$, $R_3$, $R_4$, $R_5$ or $R_6$ that is neither H nor $CH_3$ contains a halogen or is a halogen, wherein the halogen is selected from the group consisting of fluorine, chlorine, bromine and iodine. Particular embodiments of compounds of this class include compounds where a $R_1$, $R_2$, $R_3$, $R_4$, $R_5$ or $R_6$ that is neither H nor $CH_3$ is: $-NR^a-(CR^bR^c)_n-X$ wherein X is a halogen selected from the group consisting of chlorine, bromine and iodine, or is a water soluble group $R^a$, $R^b$ and $R^c$ are, independently of each other, selected from the group consisting of hydrogen and optionally substituted hydrocarbyl, and n is an integer from 0 to 20.

Preferred examples of compounds of this class are:

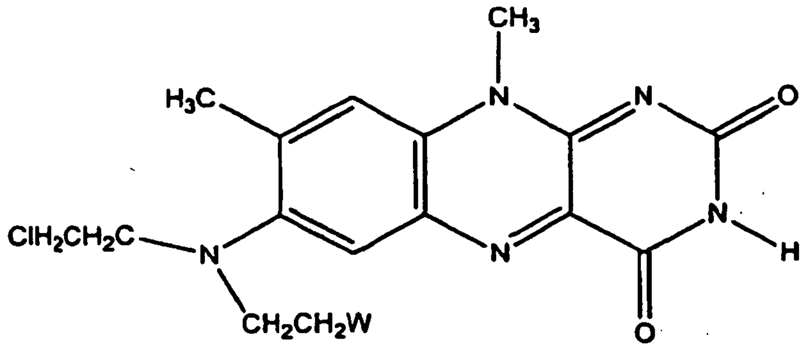

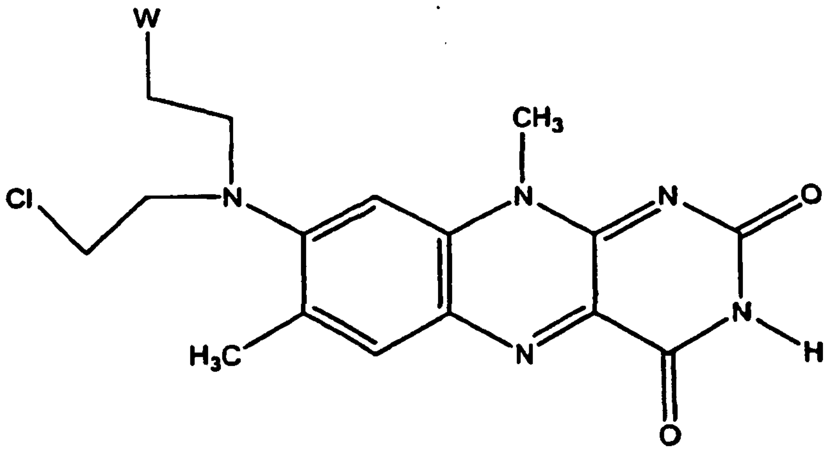

where W is a substituent imparting water solubility to the molecule, including, but not limited to, ascorbate, alcohol, polyalcohol; amine or polyamines, straight chain or cyclic saccharides, sulfates, phosphates, alkyl chains optionally substituted with -OH at any position, glycols, including polyethylene glycol and polyethers.

Another particular embodiment of compounds wherein a $R_1$, $R_2$, $R_3$, $R_4$, $R_5$ or $R_6$ that is neither H nor $CH_3$ contains a halogen or is a halogen includes compounds wherein a $R_1$, $R_2$, $R_3$, $R_4$, $R_5$ or $R_6$ that is neither H nor $CH_3$ is: $X-(CH_2)_n-$, wherein X is a halogen selected from the group consisting of chlorine, bromine and iodine, and n is an integer from 0 to 6. A preferred example of compounds of this class include:

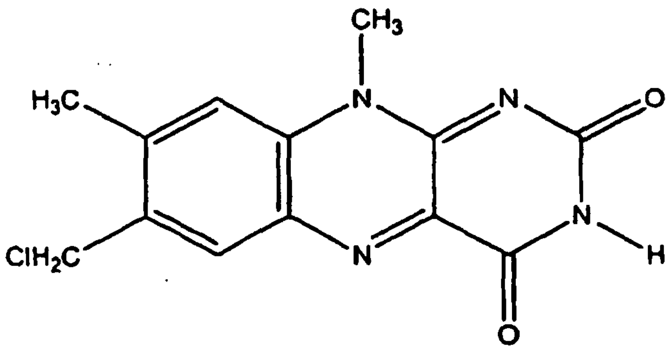

Other classes of compounds include those wherein $R_1$ is $CH_2-(CH_2OH)_3-CH_2OH$ and those wherein $R_1$ is not $CH_2(CH_2OH)_3-CH_2OH$. Also, those compounds wherein $R_3$ and $R_6$ are H are suitable for use in the invention.

A carbonyl compound is any compound containing a carbonyl group (-C=O). The term "amine" refers to a primary, secondary, or tertiary amine group. A "polyamine" is a group that contains more than one amine group. A "sulfate" group is a salt of sulfuric acid. Sulfate groups include the group $(SO4)^{2-}$. "Phosphates" contain the group $PO4^{3-}$. "Glycols" are groups that have two alcohol groups per molecule of the compound. "Glycols" are also known as diols. A

glycol is described by the formula: $C_nH_{2n}(OH)_2$, where n is an integer. An "aldehyde" is a group containing the formula -(C=O)-H. A "ketone" is a group with formula R-(C=O)-R, where R is not hydrogen. The R groups on ketone do not need to be the same. A carboxylic acid is a group which includes the formula: -COOH. An ether is a group containing -O-. A salt is a group where a hydrogen atom of an acid has been replaced with a metal atom or a positive radical, such as $NH_4^+$. Ascorbate includes groups with formula:

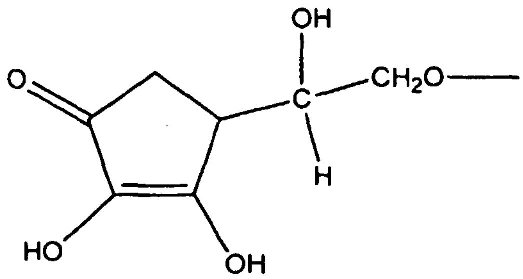

The term "hydrocarbyl" is used herein to refer generally to organic groups comprised of carbon chains to which hydrogen and optionally other elements are attached. $CH_2$ or CH groups and C atoms of the carbon chains of the hydrocarbyl may be replaced with one or more heteroatoms (i.e., non-carbon atoms). Suitable heteroatoms include but are not limited to O, S, P and N atoms. The term hydrocarbyl includes, but is not limited to alkyl, alkenyl, alkynyl, ether, polyether, thioether, straight chain or cyclic saccharides, ascorbate, aminoalkyl, hydroxylalkyl, thioalkyl, aryl and heterocyclic aryl groups, optionally substituted isoalloxazine molecules, amino acid, polyalcohol, glycol, groups which have a mixture of saturated and unsaturated bonds, carbocyclic rings and combinations of such groups. The term also includes straight-chain, branched-chain and cyclic structures or combinations thereof. Hydrocarbyl groups are optionally substituted. Hydrocarbyl substitution includes substitution at one or more carbons in the group by moieties containing heteroatoms. Suitable substituents for hydrocarbyl groups include but are not limited to halogens, including chlorine, fluorine, bromine and iodine, OH, SH, $NH_2$, COH, $CO_2H$, $OR_a$, $SR_a$, $NR_aR_b$, $CONR_aR_b$, where $R_a$ and $R_b$ independently are alkyl, unsaturated alkyl or aryl groups.

The term "alkyl" takes its usual meaning in the art and is intended to include straight-chain, branched and cycloalkyl groups. The term includes, but is not limited to, methyl, ethyl, n-propyl, isopropyl, n-butyl, sec-butyl, isobutyl, tert-butyl, n-pentyl, neopentyl, 2-methylbutyl, 1-methylbutyl, 1-ethylpropyl, 1,1-dimethylpropyl, n-hexyl, 1-methylpentyl, 2-methylpentyl, 3-methylpentyl, 4-methylpentyl, 3,3-dimethylbutyl, 2,2-dimethylbutyl, 1,1-dimethylbutyl, 2-ethylbutyl, 1-ethylbutyl, 1,3-dimethylbutyl, n-heptyl, 5-methylhexyl, 4-methylhexyl, 3-methylhexyl, 2-methylhexyl, 1-methylhexyl, 3-ethylpentyl, 2-ethylpentyl, 1-ethylpentyl, 4,4-dimethylpentyl, 3,3-dimethylpentyl, 2,2-dimethylpentyl, 1,1-dimethylpentyl, n-octyl, 6-methylheptyl, 5-methylheptyl, 4-methylheptyl, 3-methylheptyl, 2-methylheptyl, 1-methylheptyl, 1-ethylhexyl, 1-propylpentyl, 3-ethylhexyl, 5,5-dimethylhexyl, 4,4-dimethylhexyl, 2,2-diethylbutyl, 3,3-diethylbutyl, and 1-methyl-1-propylbutyl. Alkyl groups are optionally substituted. Lower alkyl groups are $C_1$-$C_6$ alkyl and include among others methyl, ethyl, n-propyl, and isopropyl groups.

The term "cycloalkyl" refers to alkyl groups having a hydrocarbon ring, particularly to those having rings of 3 to 7 carbon atoms. Cycloalkyl groups include those with alkyl group substitution on the ring. Cycloalkyl groups can include straight-chain and branched-chain portions. Cycloalkyl groups include but are not limited to cyclopropyl, cyclobutyl, cyclopentyl, cyclohexyl, cycloheptyl, cyclooctyl, and cyclononyl. Cycloalkyl groups can optionally be substituted.

Aryl groups may be substituted with one, two or more simple substituents including, but not limited to, lower alkyl, e.g., methyl, ethyl, butyl; halo, e.g., chloro, bromo; nitro; sulfato; sulfonyloxy; carboxy; carbo-lower-alkoxy, e.g., carbomethoxy, carbethoxy; amino; mono- and di-lower-alkylamino, e.g., methylamino, ethylamino, dimethylamino, methylethylamino; amido; hydroxy; lower-alkoxy, e.g., methoxy, ethoxy; and lower-alkanoyloxy, e.g., acetoxy.

The term "unsaturated alkyl" group is used herein generally to include alkyl groups in which one or more carbon-carbon single bonds have been converted to carbon-carbon double or triple bonds. The term includes alkenyl and alkynyl groups in their most general sense. The term is intended to include groups having more than one double or triple bond, or combinations of double and triple bonds. Unsaturated alkyl groups include, without limitation, unsaturated straight-chain, branched or cycloalkyl groups.. Unsaturated alkyl groups include without limitation: vinyl, allyl, propenyl, isopropenyl, butenyl, pentenyl, hexenyl, hexadienyl, heptenyl, cyclopropenyl, cyclobutenyl, cyclopentenyl, cyclopentadienyl, cyclohexenyl, cyclohexadienyl, 1-propenyl, 2-butenyl, 2-methyl-2-butenyl, ethynyl, propargyl, 3-methyl-1-pentynyl, and 2-heptynyl. Unsaturated alkyl groups can optionally be substituted.

Substitution of alkyl, cycloalkyl and unsaturated alkyl groups includes substitution at one or more carbons in the group by moieties containing heteroatoms. Suitable substituents for these groups include but are not limited to OH, SH, $NH_2$, CH, $CO_2H$, $OR_c$, $SR_c$, P, PO, $NR_cR_d$, $CONR_cR_d$, and halogens, particularly chlorines and bromines where $R_c$ and $R_d$, independently, are alkyl, unsaturated alkyl or aryl groups. Preferred alkyl and unsaturated alkyl groups are the lower alkyl, alkenyl or alkynyl groups having from 1 to about 3 carbon atoms.

The term "aryl" is used herein generally to refer to aromatic groups which have at least one ring having a conjugated pi electron system and includes without limitation carbocyclic aryl, aralkyl, heterocyclic aryl, biaryl groups and heterocyclic biaryl, all of which can be optionally substituted. Preferred aryl groups have one or two aromatic rings.

"Carbocyclic aryl" refers to aryl groups in which the aromatic ring atoms are all carbons and includes without limitation phenyl, biphenyl and napthalene groups.

"Aralkyl" refers to an alkyl group substituted with an aryl group. Suitable aralkyl groups include among others benzyl, phenethyl and picolyl, and may be optionally substituted. Aralkyl groups include those with heterocyclic and carbocyclic aromatic moieties.

"Heterocyclic aryl groups" refers to groups having at least one heterocyclic aromatic ring with from 1 to 3 heteroatoms in the ring, the remainder being carbon atoms. Suitable heteroatoms include without limitation oxygen, sulfur, and nitrogen. Heterocyclic aryl groups include among others furanyl, thienyl, pyridyl, pyrrolyl, N-alkyl pyrrolo, pyrimidyl, pyrazinyl, imidazolyl, benzofuranyl, quinolinyl, and indolyl, all optionally substituted.

"Heterocyclic biaryl" refers to heterocyclic aryls in which a phenyl group is substituted by a heterocyclic aryl group ortho, meta or para to the point of attachment of the phenyl ring to the decalin or cyclohexane. Heterocyclic biaryl includes among others groups which have a phenyl group substituted with a heterocyclic aromatic ring. The aromatic rings in the heterocyclic biaryl group can be optionally substituted.

"Biaryl" refers to carbocyclic aryl groups in which a phenyl group is substituted by a carbocyclic aryl group ortho, meta or para to the point of attachment of the phenyl ring to the decalin or cyclohexane. Biaryl groups include among others a first phenyl group substituted with a second phenyl ring ortho, meta or para to the point of attachment of the first phenyl ring to the decalin or cyclohexane structure. Para substitution is preferred. The aromatic rings in the biaryl group can be optionally substituted.

Aryl group substitution includes substitutions by non-aryl groups (excluding H) at one or more carbons or where possible at one or more heteroatoms in aromatic rings in the aryl group. Unsubstituted aryl, in contrast, refers to aryl groups in which the aromatic ring carbons are all substituted with H, e.g. unsubstituted phenyl ($-C_6H_5$), or naphthyl ($-C_{10}H_7$). Suitable substituents for aryl groups include among others, alkyl groups, unsaturated alkyl groups, halogens, OH, SH, $NH_2$, C.H., $CO_2H$, $OR_e$, $SR_e$, $NR_eR_f$, $CONR_eR_f$, where $R_e$ and $R_f$ independently are alkyl, unsaturated alkyl or aryl groups. Preferred substituents are OH, SH, $OR_e$, and $SR_e$ where $R_e$ is a lower alkyl, i.e., an alkyl group having from 1 to about 3 carbon atoms. Other preferred substituents are halogens, more preferably chlorine or bromine, and lower alkyl and unsaturated lower alkyl groups having from 1 to about 3 carbon atoms. Substituents include bridging groups between aromatic rings in the aryl group, such as $-CO_2-$, - CO-, -O-, -S-, -P-, -NH-, -CH=CH- and $-(CH_2)_P-$ where P is an integer from 1 to about 5, and particularly $-CH_2-$. Examples of aryl groups having bridging substituents include phenylbenzoate. Substituents also include moieties, such as $-(CH_2)_P-$, $-O-(CH_2)_P-$ or $-OCO-(CH_2)_P-$, where P is an integer from about 2 to 7, as appropriate for the moiety, which bridge two ring atoms in a single aromatic ring as, for example, in a 1, 2, 3, 4-tetrahydronaphthalene group. Alkyl and unsaturated alkyl substituents of aryl groups can in turn optionally be substituted as described *supra* for substituted alkyl and unsaturated alkyl groups.

The terms "alkoxy group" and "thioalkoxy group" (also known as mercaptide groups, the sulfur analog of alkoxy groups) take their generally accepted meaning. Alkoxy groups include but are not limited to methoxy, ethoxy, n-propoxy, isopropoxy, n-butoxy, sec-butoxy, isobutoxy, tert-butoxy, n-pentyloxy, neopentyloxy, 2-methylbutoxy, 1-methylbutoxy, 1-ethyl propoxy, 1,1 -dimethylpropoxy, n-hexyloxy, 1-methylpentyloxy, 2-methylpentyloxy, 3-methylpentyloxy, 4-methylpentyloxy, 3,3-dimethylbutoxy, 2,2-dimethoxybutoxy, 1-1-dimethylbutoxy, 2-ethylbutoxy, 1-ethylbutoxy, 1,3-dimethylbutoxy, n-pentyloxy, 5-methylhexyloxy, 4-methylhexyloxy, 3-methylhexyloxy, 2-methylhexyloxy, 1-methylhexyloxy, 3-ethylpentyloxy, 2-ethylpentyloxy, 1-ethylpentyloxy, 4,4-dimethylpentyloxy, 3,3-dimethylpentyloxy, 2,2-dimethylpentyloxy, 1,1-dimethylpentyloxy, n-octyloxy, 6-methylheptyloxy, 5-methylheptyloxy, 4-methylheptyloxy, 3-methylheptyloxy, 2-methylheptyloxy, 1-methylheptyloxy, 1-ethylhexyloxy, 1-propylpentyloxy, 3-ethylhexyloxy, 5,5-dimethylhexyloxy, 4,4-dimethylhexyloxy, 2,2-diethylbutoxy, 3,3-diethylbutoxy, 1-methyl-1-propylbutoxy, ethoxymethyl, n-propoxymethyl, isopropoxymethyl, sec-butoxymethyl, isobutoxymethyl, (1-ethyl propoxy)methyl, (2-ethylbutoxy)methyl, (1-ethylbutoxy)methyl, (2-ethylpentyloxy)methyl, (3-ethylpentyloxy)methyl, 2-methoxyethyl, 1-methoxyethyl, 2-ethoxyethyl, 3-methoxypropyl, 2-methoxypropyl, 1-methoxypropyl, 2-ethoxypropyl, 3-(n-propoxy)propyl, 4-methoxybutyl, 2-methoxybutyl, 4-ethoxybutyl, 2-ethoxybutyl, 5-ethoxypentyl, and 6-ethoxyhexyl. Thioalkoxy groups include but are not limited to the sulfur analogs of the alkoxy groups specifically listed *supra*.

"Optional" or "optionally" means that the subsequently described event or circumstance may or may not occur, and that the description includes instances where said event or circumstance occurs and instances in which it does not. For example, "optionally substituted phenyl" means that the phenyl radical may or may not be substituted and that the

description includes both unsubstituted phenyl radicals and phenyl radicals wherein there is substitution.

"Amino acids" as used herein include naturally occurring and commercially available amino acids and optical isomers thereof. Typical natural and commercially available amino acids are glycine, alanine, serine, homoserine, threonine, valine, norvaline, leucine, isoleucine, norleucine, aspartic acid, glutamic acid, lysine, ornithine, histidine, arginine, cysteine, homocysteine, methionine, phenylalanine, homophenylalanine, phenylglycine, o-, m-, and p-tyrosine, tryptophan, glutamine, asparagine, proline and hydroxyproline. Amino acid as used herein includes amino acid residues and amino acid side chains. An amino acid residue is an amino acid radical -NHCH(R)C(O)-, wherein R is an amino acid side chain, except for the amino acid residues of proline and hydroxyproline which are $-N(CH_2-CH_2-CH_2)CHC(O)-$ and $-N(CH-CHOHCH_2)CHC(O)-$, respectively. An amino acid side chain is a radical found on the $\alpha$-carbon of an $\alpha$-amino acid as defined herein, where the radical is either hydrogen (side chain of glycine), methyl (side chain of alanine), or is a radical bonded to the $\alpha$-carbon by a methylene ($--CH_2--$), or phenyl group.

A protected glucose derivative takes its usual meaning in the art and includes a glucose molecule wherein some of the hydroxyl groups are substituted with acetate groups.

Straight chain or cyclic saccharides include mono-, di- and poly-, straight chain and cyclic saccharides that are optionally substituted with an amino group which is optionally acetylated. Straight chain saccharides that are useful in this invention include but are not limited to those molecules with a chain of 5 or 6 carbon atoms with one or more -OH groups attached, and either an aldehyde or ketone group. Cyclic saccharides are saccharides that are in a ring form. Disaccharides are compounds wherein two monosaccharide groups are linked. Polysaccharides are compounds wherein more than two monosaccharide groups are linked. Specific examples of saccharides useful in this invention include glucose, ribose and glucosamine, among others.

Isoalloxazine, isoalloxazine derivative or core structure of isoalloxazine include compounds that comprise the structure:

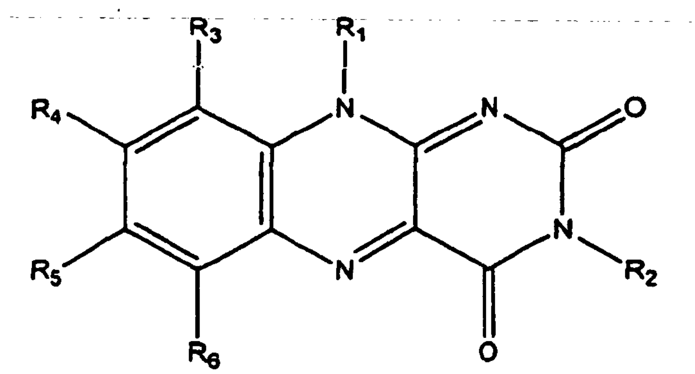

where $R_1$-$R_6$ are substituted with various substituents, as described elsewhere.

As used herein, the term neutralization of a microorganism or neutralizing means totally or partially preventing the microorganism from replicating, either by killing the microorganism or otherwise interfering with its ability to reproduce. A neutralizer is a compound that is capable of neutralizing a microorganism. The neutralizers useful in this invention include molecules with the core structure of isoalloxazine, as defined above. To activate the microorganism neutralizer is to expose the microorganism neutralizer to a triggering event that causes it to become active toward neutralizing microorganisms.

Triggering event refers to the stimulus that activates the microorganism neutralizer. The triggering event used in the method of the present invention is exposure of the neutralizer to a neutralization effective wavelength of light.

Water soluble group includes a group that, when included as a substituent on the neutralizer, imparts substantial solubility in water to the compound. Typically, the compound is soluble in water at a concentration of about 10 - 150 $\mu$M. Water soluble groups as referred to in this invention include, but are not limited to alcohols; polyalcohols; straight chain or cyclic saccharides; amines and polyamines; sulfate groups; phosphate groups; ascorbate groups; alkyl chains optionally substituted with - OH at any position; glycols, including polyethylene glycols, and polyethers.

Decomposition of the neutralizer upon exposure to light refers to the chemical transformation of the neutralizer into new compounds. An example of decomposition of the neutralizer is the production of lumichrome upon exposure of riboflavin to visible light.

An "alkylating agent" is a compound that reacts with amino acid residues and nucleic bases and inhibits replication of microorganisms.

The terms "photoactivator" and "photosensitizer" are used synonymously herein.

As used herein, "powder" means dried medium, including powder or pill. When a dried medium of a substance is described herein, it is also intended that a solution or suspension of the powder in a suitable solvent may be used, and vice versa.

The method of this invention requires mixing the photosensitizer with the material to be decontaminated. "Adding" is intended to include mixing the fluid with the photosensitizer. Mixing may be done by simply adding the photosensitizer or a solution containing the photosensitizer to a fluid to be decontaminated. In one embodiment, the material to be decontaminated to which photosensitizer has been added is flowed past a photoradiation source, and the flow of the material generally provides sufficient turbulence to distribute the photosensitizer throughout the fluid to be decontaminated. In another embodiment, the fluid and photosensitizer are placed in a photopermeable container and irradiated in batch mode, preferably while agitating the container to fully distribute the photosensitizer and expose all the fluid to the radiation.

The amount of photosensitizer to be mixed with the fluid will be an amount sufficient to adequately inactivate microorganisms therein, but less than a toxic (to humans or other mammals) or insoluble amount. Excess photosensitizer may be used as long as the concentration is not so high that the photosensitizer prevents light from passing to the desired depth at a useful intensity. As taught herein, optimal concentrations for desired photosensitizers may be readily determined by those skilled in the art without undue experimentation. Preferably the photosensitizer is used in a concentration of at least about 1 micromolar. The optimum concentration of photosensitizer will vary depending on the blood component being treated and if plasma is removed, as discussed herein. If red blood cells are being treated, a higher concentration of photosensitizer is desired than if plasma or platelets are being treated. If red blood cells are being treated with riboflavin, a useful concentration of riboflavin is about 1-500 micromolar, including all values and ranges therein, including 1 to 200 micromolar. A preferred concentration of riboflavin is about 300 to 500 micromolar, particularly when the plasma content is about 0 to 5% of the total volume of the solution. If plasma or platelets are being treated, a useful concentration of riboflavin is about 1-100 micromolar, and a preferred concentration of riboflavin is about 10 to 50 micromolar, including all values and ranges therein, including 10 to 30 micromolar when the plasma content is about 10 - 90% of the total volume of the solution.

The activated photosensitizer is capable of inactivating the microorganisms present, such as by interfering to prevent their replication. Specificity of action of the photosensitizer is conferred by the close proximity of the photosensitizer to the nucleic acid of the microorganism and this may result from binding of the photosensitizer to the nucleic acid. Nucleic acid includes ribonucleic acid (RNA) and deoxyribonucleic acid (DNA). Other photosensitizers may act by binding to cell membranes or by other mechanisms. The photosensitizer may also be targeted to the microorganism to be inactivated by covalently coupling to an antibody, preferably a specific monoclonal antibody to the microorganism.

The fluid containing the photosensitizer may be flowed into a photopermeable container for irradiation. The term container refers to a closed or open space, which may be made of rigid or flexible material, e.g., may be a bag or box or trough. It may be closed or open at the top and may have openings at both ends, e.g., may be a tube or tubing, to allow for flow-through of fluid therein. A cuvette has been used to exemplify one embodiment of the invention involving a flow-through system. Collection bags, such as those used with the Trima™ Spectra™ and apheresis systems of GAMBRO, Inc., have been used to exemplify another embodiment involving batch-wise treatment of the fluid.

The term photopermeable means the material of the container is adequately transparent to photoradiation of the proper wavelength for activating the photosensitizer. In the flow-through system, the container has a depth (dimension measured in the direction of the radiation from the photoradiation source) sufficient to allow photoradiation to adequately penetrate the container to contact photosensitizer molecules at all distances from the light source and ensure inactivation of microorganisms in the fluid to be decontaminated, and a length (dimension in the direction of fluid flow) sufficient to ensure a sufficient exposure time of the fluid to the photoradiation. The materials for making such containers, depths and lengths of containers may be easily determined by those skilled in the art without undue experimentation following the teachings herein, and together with the flow rate of fluid through the container, the intensity of the photoradiation and the absorptivities of the fluid components, e.g., plasma, platelets, red blood cells, will determine the amount of time the fluid needs to be exposed to photoradiation.

In another embodiment involving batch-wise treatment, the fluid to be treated is placed in a photopermeable container which is agitated and exposed to photoradiation for a time sufficient to substantially inactivate the microorganisms. The photopermeable container is preferably a blood bag made of transparent or semitransparent plastic, and the agitating means is preferably a shaker table. The photopermeable container may be any other container, such as a rigid plastic container. The photosensitizer may be added to the container in powdered or liquid form and the container agitated to mix the photosensitizer with the fluid and to adequately expose all the fluid to the photoradiation to ensure inactivation of microorganisms.

Photosensitizer may be added to or flowed into the photopermeable container separately from the fluid being treated or may be added to the fluid prior to placing the fluid in the container. In one embodiment, photosensitizer is added to anticoagulant and the mixture of photosensitizer and anticoagulant are added to the fluid.

After treatment, the blood or blood product may be delivered to a patient, concentrated, or infused directly.

Enhancers may also be added to the fluid to make the process more efficient and selective. Such enhancers include antioxidants or other agents to prevent damage to desired fluid components or to improve the rate of inactivation of microorganisms and are exemplified by adenine, histidine, cysteine, tyrosine, tryptophan, ascorbate, N-acetyl-L-cysteine, propyl gallate, glutathione, mercaptopropionylglycine, dithiothreotol, nicotinamide, BHT, BHA, lysine, serine, methionine, glucose, mannitol, trolox, glycerol, and mixtures thereof. These enhancers may be added in dried medium, including powder or pill form or in the form of liquids.

Use of the method of the invention leads to fluids comprising human whole blood, a human blood constituent or an aqueous composition containing biologically active protein derived from human blood, inactivated *P. Falciparum*; and endogenous alloxazine or endogenously-based derivative alloxazine or a photoproduct of said alloxazine. The fluid may also contain inactivated microorganism.

Typically, the fluid is a blood product comprising inactivated *P. falciparum* and endogenous alloxazine or endogenously-based derivative alloxazine or a photoproduct of said alloxazine.

Preferably, the fluid is one comprising red blood cells, inactivated *P. falciparum* and 7,8-dimethyl-10-ribityl isoalloxazine or a photoproduct of said 7,8-dimethyl-10-ribityl isoalloxazine.

In decontamination systems used in the present invention, the photoradiation source may be connected to the photopermeable container for the fluid by means of a light guide such as a light channel or fiber optic tube which prevents scattering of the light between the source and the container for the fluid, and more importantly, prevents substantial heating of the fluid within the container. Direct exposure to the light source may raise temperatures as much as 10 to 15°C, especially when the amount of fluid exposed to the light is small, which can cause denaturization of blood components. Use of the light guide keeps any heating to less than about 2°C. The method may also include the use of temperature sensors and cooling mechanisms where necessary to keep the temperature below temperatures at which desired proteins in the fluid are damaged. Cooling mechanisms include flow of air or fluid, as well as other mechanisms known to the art. Preferably, the temperature is kept between about 0° and about 45°C, more preferably between about 22° C and about 45°C, and preferably about 30°C, depending on the composition of the fluid. The heating of fluids from light exposure is known in the art and conditions to prevent damage to desired components of the fluids are known in the art without undue experimentation and described in the references cited herein. Usual operating temperature ranges for red blood cells are 32 to 36°C and for platelets and plasma, below 30°C.

Any means for adding the photosensitizer to the fluid to be decontaminated and for placing the fluid in the photopermeable container known to the art may be used, such means typically including flow conduits, ports, reservoirs, sterile docking and valves. The system may include means such as pumps or adjustable valves for controlling the flow of the photosensitizer into the fluid to be decontaminated so that its concentration may be controlled at effective levels as described herein. In one embodiment, photosensitizer is mixed with the anticoagulant feed to a blood apheresis system. For endogenous photosensitizers and derivatives having sugar moieties, the pH of the solution is preferably kept low enough, as is known to the art, to prevent detachment of the sugar moiety. Preferably the photosensitizer is added to the fluid to be decontaminated in a pre-mixed aqueous solution, e.g., in water or storage buffer solution.

The photosensitizer and any optional desired additives may be placed in a container as dried medium, including powder or pill form, or as a solution. Optional additives may be chosen that help the components retain biological activity or improve the storage lifetime. These optional additives are known in the art. Desired additives and the photosensitizer may be sterilized as powders. In one embodiment, the powders desired are placed in the container prior to introduction of the fluid.

The level of plasma in the solutions may be adjusted, if desired. If the photosensitizer and any desired additives are placed in the container as one or more solutions, the volume and composition of the solution(s) may produce the desired percentage of plasma in the sample, without further additions of solution, or the percentage of plasma may be adjusted before, during or after placing said fluid in said container. Adjustment of the percentage of plasma after placing the fluid in the container may occur by the introduction of a suitable solution after the fluid is in the container. Adjustment of the percentage of plasma may occur during introduction of the fluid in a container by the introduction of a suitable solution as the fluid is being placed in the container.

The photopermeable container for the flow-through system may be a transparent cuvette made of polycarbonate, glass, quartz, polystyrene, polyvinyl chloride, polyolefin, or other transparent material. The cuvette may be enclosed in a radiation chamber having mirrored walls. A photoradiation enhancer such as a second photoradiation source or reflective surface may be placed adjacent to the cuvette to increase the amount of photoradiation contacting the fluid within the cuvette. The system preferably includes a pump for adjusting the flow rate of the fluid to desired levels to ensure substantial decontamination as described above. The cuvette has a length, coordinated with the flow rate therethrough, sufficient to expose fluid therein to sufficient photoradiation to effect substantial decontamination thereof.

Also preferably the cuvette is spaced apart from the light source a sufficient distance that heating of the fluid in the cuvette does not occur, and light is transmitted from the light source to the cuvette by means of a light guide.

In another embodiment the fluid is placed in a photopermeable container such as a blood bag, e.g. used with the apheresis system described in U.S. Patent No. 5,653,887, and agitated while exposing to photoradiation. A photoradiation enhancer such as a second photoradiation source or reflective surface may be placed adjacent to the cuvette to increase the amount of photoradiation contacting the fluid within the cuvette. Suitable bags include collection bags as described herein. Collection bags used in the Cobe Spectra™ system or Trima™ apheresis system of GAMBRO Inc. are especially suitable. Shaker tables are known to the art, e.g. as described in U.S. Patent 4,880,788. The bag is equipped with at least one port or opening for adding fluid thereto. In one embodiment the photosensitizer, preferably 7,8-dimethyl-10-ribityl-isoalloxazine, is added to the fluid-filled bag as dried medium, including powder or pill form. The bag is then placed on a shaker table and agitated under photoradiation until substantially all the fluid has been exposed to the photoradiation. Alternatively, the bag may be prepackaged with the powdered photosensitizer contained therein. The fluid to be decontaminated may then be added through the appropriate port.

Decontamination systems as described above may be designed as stand-alone units or may be easily incorporated into existing apparatuses known to the art for separating or treating blood being withdrawn from or administered to a patient. For example, such blood-handling apparatuses include the Cobe Spectra™ or GAMBRO TRIMA™ apheresis systems, available from GAMBRO Inc., Lakewood, CO, or the apparatuses described in U.S. Patent 5,653,887 and U.S. Serial No. 08/924,519 filed September 5, 1997 (PCT Publication No. WO 99/11305) of GAMBRO, Inc. as well as the apheresis systems of other manufacturers. The decontamination system may be inserted just downstream of the point where blood is withdrawn from a patient or donor, just prior to insertion of blood product into a patient, or at any point before or after separation of blood constituents. The level of plasma may be adjusted, if desired, at any point before fluid is exposed to irradiation. The photosensitizer is added to blood components along with anticoagulant in a preferred embodiment, and separate irradiation sources and cuvettes are placed downstream from collection points for platelets, for plasma and for red blood cells. The use of three separate blood decontamination systems is preferred to placement of a single blood decontamination system upstream of the blood separation vessel of an apheresis system because the lower flow rates in the separate component lines allows greater ease of irradiation. In other embodiments, decontamination systems of this invention may be used to process previously collected and stored blood products.

When red blood cells are present in the fluid being treated, as will be appreciated by those skilled in the art, to compensate for absorption of light by the cells, the fluid may be thinned, exposed to higher energies of radiation for longer periods, agitated for longer periods or presented to photoradiation in shallower containers or conduits than necessary for use with other blood components.

The endogenous alloxazine photosensitizers and endogenously-based derivative alloxazine photosensitizers disclosed herein can be used in pre-existing blood component decontamination systems as well as in the decontamination system disclosed herein. For example, the endogenous alloxazine photosensitizers and endogenously-based derivative alloxazine photosensitizers of this invention can be used in the decontamination systems described in U.S. Patent Nos. 5,290,221, 5,536,238, 5,290,221 and 5,536,238.

A photoradiation enhancer, such as a reflective surface may also be provided in any method or apparatus of the invention. The light may be guided to impinge on the fluid in any desired manner, including positioning the fluid in the light path of the light source, using a light guide, or other methods. The apparatuses of the invention may also comprise components such as a temperature monitor, temperature controller, means for flowing said fluid into and out of said container, means for agitating said fluid in said container, and other desired components to control various aspects of the system. The temperature controller may be a fan directed toward the light source, directed on the fluid, or both. One or more temperature controllers may be used to cool different components to different levels.

The methods of the invention may be used in a kit, for example, a kit to determine if a virus or parasite (or other microorganism) is present in blood or a blood component. The fluid of the present invention is typically a component of a kit. For example, one kit includes a sample of blood or a blood component that has been inactivated by the methods of the invention. This inactivated sample is compared to a non-inactivated sample to determine if a virus or parasite (or other microorganism) is present in the blood or a blood component or other sample.

Figure 1 shows *P. falciparum* inactivation results from 38% Hct red blood cell samples with no incubation prior to illumination.

Figure 2 shows *P. falciparum* inactivation results from illuminating the innoculum (no red blood cells).

Figure 3 shows *P. falciparum* inactivation results from illuminating 38% Hct red blood cell suspension that was incubated for 1 hour prior to illumination.

The following applications are hereby referenced to the extent not inconsistent with the disclosure herewith: United States patent application serial no. 10/104,766, filed March 21, 2002; United States patent application serial no. 10/247,262 filed September 18, 2002; United States provisional application serial no. 60/368,778, filed March 28, 2002; United States patent application serial no. 10/159,781, filed May 30, 2002; United States patent application serial no. 09/982,298, filed October 16, 2001; United States patent application serial no. 10/328,717, filed December 23, 2002; United States patent application serial no. 10/065,073, filed September 13, 2002; United States patent application serial no. 09/962,029, filed September 25, 2001; United States provisional application serial no. 60/353,223, filed February 1, 2002; United States provisional application serial no. 60/355,393, filed February 8, 2002; United States provisional application serial no. 60/377,697, filed May 3, 2002; United States patent application serial no. 10/325,402, filed December

20, 2002 (attorney docket no. B0109-US02); United States provisional application serial no. 60/353,319, filed February 1, 2002; United States provisional application serial no. 60/379,328, filed May 8, 2002; United States provisional application serial no. 60/375,734, filed April 26, 2002; United States provisional application serial no. 60/373,198, filed April 16, 2002; United States provisional application serial no. 60/373,936, filed April 19, 2002; United States provisional application serial no. 60/378,374, filed May 6, 2002; United States provisional application serial no. 60/375,849, filed April 24, 2002; United States patent application serial no. 09/586,147, filed June 2, 2000; United States patent application serial no. 09/596,429, filed June 15, 2000; United States provisional application serial no. 60/375,670, filed April 26, 2002; PCT patent application serial no. PCT/US02/21925, filed July 12, 2002; United States provisional application serial no. 60/319,488, filed August 23, 2002; United States provisional application serial no. 60/319,641, filed October 22, 2002; United States patent application serial no. 09/119,666, filed July 21, 1998 (United States patent 6,258,577); United States patent application serial no. 09/357,188, filed July 20, 1999 (United States patent 6,277,337); United States patent application serial no. 09/420,652, filed October 19, 1999 (United States patent 6,268,120); United States patent application serial no. 09/777,727, filed February 5, 2001; United States patent application serial no. 10/256,852, filed September 26, 2002; United States patent application serial no. 09/725,426, filed November 28, 2000. For example, apparatuses and systems other than those specifically exemplified in the disclosure herewith are included in the cited applications and patents.

The decontamination method of this invention using endogenous alloxazine photosensitizers and endogenously-based derivative alloxazine photosensitizers is exemplified herein using 7,8-dimethyl-10-ribityl isoalloxazine as the photosensitizer, however, any such photosensitizer may be used which is capable of being activated by photoradiation to cause inactivation of microorganisms. The photosensitizer must be one which does not substantially destroy desired components of the fluid being decontaminated, and also preferably which does not break down as a result of the photoradiation into products which significantly destroy desired components or have significant toxicity. The wavelength at which the photosensitizer is activated is determined as described herein, using literature sources or direct measurement. Its solubility in the fluid to be decontaminated or in a combination of carrier fluid and fluid to be contaminated is also so determined. The ability of photoradiation at the activating wavelength to penetrate the fluid to be decontaminated is also determined as taught herein and known in the art. Appropriate temperatures for the reaction of the photosensitizer with its substrate are determined, as well as the ranges of temperature, photoradiation intensity and duration and photosensitizer concentration which will optimize microbial inactivation and minimize damage to desired proteins and/or cellular components in the fluid.

Once such system requirements have been determined for flow-through systems, apparatuses may be designed which provide the correct flow rates, photopermeabilities, plasma contents, light wavelengths and light intensities to cause inactivation of microorganisms present in the fluid, as is taught herein. In one embodiment, the fluid is mixed with photosensitizer and then irradiated with a sufficient amount of photoradiation to activate the photosensitizer to react with microorganisms in the fluid such that microorganisms in the fluid are inactivated. The amount of photoradiation reaching microorganisms in the fluid is controlled by selecting an appropriate photoradiation source, an appropriate distance of the photoradiation source from the fluid to be decontaminated, which may be increased through the use of light guides to carry the photoradiation directly to the container for the fluid, an appropriate photopermeable material for the container for the fluid, an appropriate depth to allow full penetration of the photoradiation into the container, photoradiation enhancers such as one or more additional photoradiation sources, preferably on the opposite side of the container from the first, or reflectors to reflect light from the radiation source back into the container, appropriate flow rates for the fluid in the container and an appropriate container length to allow sufficient time for inactivation of microorganisms present. Temperature monitors and controllers may also be required to keep the fluid at optimal temperature.

For batch systems, it is preferred to place the fluid to be decontaminated along with photosensitizer in bags which are photopermeable or at least sufficiently photopermeable to allow sufficient radiation to reach their contents to activate the photosensitizer. Sufficient photosensitizer is added to each bag to provide inactivation, and the bag is preferably agitated while irradiating, for a period of time to ensure exposure of substantially all the fluid to radiation. The photosensitizer may be added in powdered form.

The method preferably uses endogenous alloxazine photosensitizers, including endogenous alloxazine photosensitizers which function by interfering with nucleic acid replication. 7,8-dimethyl-10-ribityl isoalloxazine is the preferred photosensitizer for use in this invention. The chemistry believed to occur between 7,8-dimethyl-10-ribityl isoalloxazine and nucleic acids does not proceed via singlet oxygen-dependent processes (i.e. Type II mechanism), but rather by direct sensitizer-substrate interactions (Type 1 mechanisms). Cadet et al. (1983) J. Chem., 23:420-429, clearly demonstrate the effects of 7,8-dimethyl-10-ribityl isoalloxazine are due to non-singlet oxygen oxidation of guanosine residues. In addition, adenosine bases appear to be sensitive to the effects of 7,8-dimethyl-10-ribityl isoalloxazine plus UV light. This is important since adenosine residues are relatively insensitive to singlet oxygen-dependent processes. 7,8-dimethyl-10-ribityl isoalloxazine appears not to produce large quantities of singlet oxygen upon exposure to UV light, but rather exerts its effects through direct interactions with substrate (e.g., nucleic acids) through electron transfer reactions with excited state sensitizer species. Since indiscriminate damage to cells and proteins arises primarily from singlet oxygen

sources, this mechanistic pathway for the action of 7,8-dimethyl-10-ribityl isoalloxazine allows greater selectivity in its action than is the case with compounds such as psoralens which possess significant Type II chemistry.

7,8-dimethyl-10-ribityl isoalloxazine (Riboflavine or vitamin B2) absorbs light from about 200 to 500 nm. The ring system core of 7,8-dimethyl-10-fibityl isoalloxazine is resistant to photodegradation but the ribityl side chain of riboflavin undergoes photodegradation. Photolysis of 7,8-dimethyl-10-ribityl isoalloxazine may form lumichrome (7,8-dimethylalloxazine) depending on conditions. 7,8-dimethylalloxazine strongly absorbs ultraviolet (UV) light and only weakly absorbs visible light.

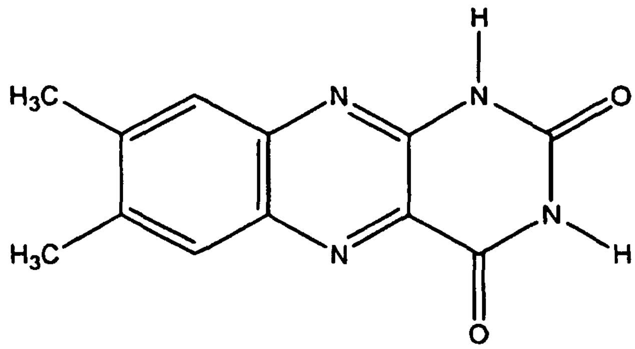

**7,8-dimethylalloxazine**

As a result of the degradation of 7,8-dimethyl-10-ribityl isoalloxazine upon exposure to light, a combination of visible and ultraviolet light is preferred in decontamination procedures using 7,8-dimethyl-10-ribityl isoalloxazine. Since UV light has a higher energy per photon than visible light, and because UV light is absorbed more strongly than visible light by useful compounds in the biological fluid, more damage to the useful components in the biological fluid containing the contaminants will occur when ultraviolet light is used in combination with visible light than when visible light can be used alone.

The methods of this invention do not require the use of enhancers such as quenchers or oxygen scavengers, however these may be used to enhance the process by reducing the extent of non-specific cell or protein-damaging chemistry or enhancing the rate of pathogen inactivation. Further preferred methods using non-toxic endogenous photosensitizers and endogenously-based derivative photosensitizers do not require removal of photosensitizers from the fluid after photoradiation.

Apparatus design.

The methods of the invention may be used in a variety of devices. The devices generally comprise: a light source producing light having sufficient wavelength and power to induce inactivation of microorganisms which may be present in a sample; and means for positioning the sample so that it receives energy of sufficient wavelength and power to induce inactivation of microorganisms.

The system preferably includes means for producing movement in the sample. Movement provides many benefits including improving the efficiency of the inactivation reactions by helping mix the photosensitizer with the fluid to be deactivated and providing turnover of sample at the container-light interface, for example. An agitator, such as a Helmer flatbed agitation system (Helmer) may be used. This agitator provides oscillatory motion. Other types of agitators may be used to provide motion normal to the bag. If a bag is used as a container, in combination with a source of movement, a pin or other structure may be placed across or within the bag to provide turbulent eddies in the fluid. The agitator may be connected to a computer or other controller in an inactivation system. Some parameters that may be controlled or monitored include temperature of the fluid, energy output of the lights, agitation motion, light control, timing control or monitoring, and other parameters. The light source and fluid being treated may both move to provide agitation of the fluid, or only the fluid being treated may move while the light source remains stationary.

One particular embodiment of the apparatus is an enclosed photoradiation system where the sample would be placed in an apparatus similar to the Bio-Genic irradiator (Vilber-Lourmat, Cedex, France) that uses the appropriate wavelength or wavelengths. Another embodiment is a conveying apparatus used in a large-scale operation to carry samples through a light field or series of light fields.

Means for positioning the sample so that it receives energy of sufficient wavelength and power to induce inactivation of microorganisms include a shelf or tray for the sample to be disposed upon; a gap between two supports

which may be a light or light arrays, where the sample is positioned between the supports; or other means as known in the art. The shelf or tray may move, as in a conveyer line. Fluid-holding shelves may be transparent to one or more of the wavelength(s) of light applied.

The sample may be placed in a suitable container on a support surface between two or more sources of photoradiation, like a sandwich. Alternatively, one of the photoradiation-sources may be a reflective material, to allow the light to contact both sides of the sample. Alternatively, or in combination, the sample may be placed on a support and light may impinge on one surface, with agitation, to allow different portions of the sample to be in contact with the light.

Different sources of photoradiation may be used, depending on the wavelength desired and the power desired at the desired wavelength. One light source that may be used has an emission centered around 447 nm.

Lights that emit in the blue spectral range come from various sources. Lamps with peak emissions around 420 to 450 nm may be purchased from LCD Lighting, Orange, CT; Bulbtronic, Farmingdale, NY; National Biological Corp., Twinsburg, OH; The Fluorescent Co., Saugus, CA; Tek-West, Los Angeles, CA; or Southern NE UV, Bransford, CT, for example. LED (light emitting diodes) may also be used. These LEDs may use a variety of materials to produce the desired spectral output, including silicon carbide (bandwidth around 100 nm; peak spectral output near 466nm) or gallium nitride (bandwidth around 30 - 35 nm; peak spectral output near 470nm). Also, lights made from a combination of different materials can generate different wavelengths of light. For example, gallium nitride on a silicon carbide substrate can generate 430 nm. These LEDs are manufactured or distributed by Panasonic, Chicago Miniature, Nichia, Toyoda Gosei, Hewlett Packard, LEDTronics, for example. LED lights typically do not require any outside cooling.

The lights may be used in different ways, depending the particular apparatus. For example, diodes may be duty cycled to emit light when the sample arrives in a flow cell light path. Arrays of diodes may surround the fluid in any desired configuration. In a flat bed apparatus, light arrays may surround the fluid from top or bottom, or both.

Filters, such as color glass filters, may be used to isolate a desired band of the spectrum. Single wavelength or narrow band sources may also be used.

One embodiment of an apparatus useful in the methods of the invention includes banks of interchangeable lights that produce the desired wavelength of light for the particular fluid being treated. A coral or aquarium light may be used to produce wavelengths between 440 and 470 nm that is useful in inactivating microorganisms in red blood cells. The lamps may be provided with separate power supplies to control the level of light output. These lamps may be sequentially placed in position to impinge light on the sample, or the sample may travel through lights of different wavelengths. Different LEDs emitting each desired wavelength may be combined in one array.

Active (cooling through some applied means) or passive (air cooling) cooling may be used if necessary to cool either the lamps or the blood. Fans may provide cooling. One set of fans may be used to cool both the lamps and blood, or different fans may be used to provide different levels of cooling to both the lamps and the blood. A photopermeable fluid may surround the sample and/or lights to provide active cooling. This fluid may be optionally temperature controlled. *Plasmodium falciparum* in other blood components and with other regions of light proceeds analogously, and is well within the skill of one of ordinary skill in the art using the teachings herein. Effectiveness of the method was evaluated with measurements of parasite viability in treated units compared to that in controls. Infected red cells were treated at two different hematocrits: 6%, with 4% of the red cells infected, and 38%, with 0.4% of the red cells infected. In each test, the parasite viability decreased to background levels during post-treatment incubation, indicating that riboflavin and light inactivate *P. falciparum*.

Parasite Inoculum:

*Plasmodium falciparum* parasite (strain NF54 [Ponnudurai T, et al. The production of mature gametocytes of Plasmodium falciparum in continuous culture of different isolates infective to mosquitoes. Trans Roy Soc Trop Med Hyg. 1982;76:242]) was cultivated from a continuous stock culture (modified Trager and Jensen technique [Trager W, Jensen JB. Human malaria parasites in continuous culture. Science. 1976;193:673]) maintained at the Walter Reed Army Institute of Research Malaria Culture Laboratory. The inoculum was prepared with 5 mL of stock culture, 95 mL of RPMI media, and 6% human red blood cells (RBCs) and incubated at 37°C and 100rpm after brief exposure to 5% $CO_2$, 5% $O_2$, and 90% $N_2$ gas. The inoculum media was changed daily when the parasitemia was below 5%, and twice daily for parasitemia greater than 5%.

Donor Red Blood Cells

Blood from volunteer donors was collected into citrate-phosphate-dextrose (CPD). The donor whole blood units were centrifuged at 5,000g for 10 minutes; after centrifugation, plasma and some of the buffy-coat was removed and discarded, leaving packed RBCs (pRBCs).

Preparation for Illumination

The parasite inoculum was centrifuged and the supernatant was removed.
For Illumination of Inoculum only:

The parasite inoculum was centrifuged and the supernatant was removed. For Illumination of Inoculum only:

-- 16 mL of parasite inoculum and 24 mL of 500 μM riboflavin solution added to 150-mL PVC bag with 10 mL of air for a Hct = 6% and parasitemia = 4%
-- Incubation for 1 hour at room temperature with mixing prior to illumination

For Illumination of Red Cells at 38% Hct:

-- 10 mL of pRBCs combined with 6 mL parasitized inoculum and 500 μM riboflavin solution to volume of 40 mL with Hct = 38% and parasitemia = 0.4%
-- One set of tests at 38% Hct involved illumination directly after preparation of the suspension (No Incubation); the other set of tests at 38% Hct involved incubation for 1-hr at room temperature with mixing prior to illumination (With Incubation).

Samples of 0.3 mL were removed from all suspensions before illumination.

Illumination

After preparation, and incubation with the riboflavin solution, red cell suspensions were illuminated with 450 nm light for 30, 45, or 60 minutes in 150 mL DEHP bags, CharterMed.
Energy delivered was 60, 90, and 120 J/cm$^2$, respectively.
•After illumination, 1-mL samples were removed from each suspension.

Sample preparation and analysis

Parasite viability was examined by measuring parasitic lactate dehydrogenase (pLDH) levels with a double monoclonal antibody ELISA technique (Druilhe, P., et al. A colorimetric in vitro drug-sensitivity assay for P. falciparum based on a highly sensitive two-site LDH antigen capture ELISA assay. Am J Trop Med Hyg 2001 May-Jun;64:233-41). Measurements of pLDH correlate linearly with % parasitemia. Viability of the parasite is reflected in pLDH. Samples removed prior to illumination were prepared for the assay and stored immediately. Samples removed after illumination were incubated for 2 weeks in multi-well plates with RPMI and aliquots were periodically removed, prepared for the assay, and stored. At the end of the 2-week incubation, all samples were assayed for pLDH content. % Parasitemia of the parasitic inoculum was determined from counts of infected RBCs per total number of RBCs; this value and the pLDH values for untreated inoculum and uninoculated RBCs were used to determine a proportionality constant relating pLDH to % parasitemia:

$$\kappa = \%\ \text{Parasitemia of Inoculum} / (\text{pLDH}_{\text{inoculum}} - \text{pLDH}_{\text{uninoculatedRBCs}})$$

The proportionality constant, κ, is used to calculate % Parasitemia for all samples:

$$\%\ \text{Parasitemia of Sample} = \kappa * (\text{pLDH}_{\text{sample}} - \text{pLDH}_{\text{uninoculatedRBCs}})$$

Results

Figure 1 shows inactivation results from 38% Hct RBC samples with no incubation prior to illumination. Figure 2 shows inactivation results from illuminating the innoculum (no RBCs). Figure 3 shows inactivation results from illuminating 38% Hct RBC suspension that was incubated for 1 hour prior to illumination.
The results indicate that riboflavin and visible light significantly reduce parasite viability. Treatment of low Hct inoculum yielded most rapid reduction in parasite viability. Illumination of red cell suspension after 1 hour of incubation

with riboflavin solution yielded more rapid reduction in parasite viability than illumination without incubation. Unilluminated controls with riboflavin solution exhibited decreased parasite viability over short incubation times; over longer times, parasite viability recovered.

## Claims

1. A method for treating a fluid to inactivate at least the parasite *P. falciparum* that may be present therein, said fluid containing one or more components selected from human whole blood, human blood constituents, or aqueous compositions containing biologically active protein derived from human blood, the method comprising:

   (a) adding an inactivation effective, substantially non-toxic amount of a photosensitizer which is an endogenous alloxazine or endogenously-based derivative alloxazine to the fluid; and
   (b) exposing the fluid of step (a) to photoradiation sufficient to activate the photosensitizer, whereby at least any *P. falciparum* present in the fluid is inactivated.

2. The method of claim 1, wherein the photosensitizer is an endogenous alloxazine.

3. The method of claim 2, wherein the photosensitizer is 7,8-dimethyl-10-ribityl isoalloxazine.

4. The method of claim 1, wherein the photoradiation comprises one or more wavelengths in the visible spectrum.

5. The method of claim 1, wherein the photoradiation comprises one or more wavelengths in the ultraviolet spectrum.

6. The method of claim 1, wherein said fluid of step (a) is exposed to a source of photoradiation at a depth selected to ensure penetration of the photoradiation through the fluid and inactivation of the parasite.

7. The method of claim 1, wherein the fluid comprises human blood constituents.

8. The method of claim 1, wherein the fluid is human whole blood.

9. The method of claim 1, wherein the fluid is a separated human blood product.

10. The method of claim 1, wherein an endogenous photosensitizer is added to anticoagulant and said anticoagulant is added to the fluid.

11. The method of claim 1 wherein a pre-mixed aqueous solution comprising the photosensitizer is added to the fluid.

12. The method of claim 3 wherein the photosensitizer is added to the fluid at a concentration of 300-500 $\mu$M.

13. The method of claim 1 further comprising the additional step of adjusting the level of plasma in the fluid.

## Patentansprüche

1. Verfahren zur Behandlung eines Fluids zur Inaktivierung von mindestens dem Parasiten *P. falciparum*, der darin vorhanden sein kann, wobei das Fluid eine oder mehr Komponenten, ausgewählt aus menschlichem Vollblut, menschlichen Blutbestandteilen oder wässrigen Zusammensetzungen, welche biologisch aktives Protein, abgeleitet von menschlichem Blut, enthalten, enthält, wobei das Verfahren umfasst:

   (a) das Zugeben einer wirksamen, im Wesentlichen nicht toxischen Inaktivierungsmenge eines Photosensibilisators, welcher ein endogenes Alloxazin oder Alloxazinderivat auf endogener Basis ist, zu dem Fluid; und
   (b) das Einwirken von Photostrahlung, welche zur Aktivierung des Photosensibilisators ausreichend ist, auf das Fluid von Schritt (a), wobei mindestens jedweder in dem Fluid vorhandene *P. flaciparum* inaktiviert wird.

2. Verfahren nach Anspruch 1, wobei der Photosensibilisator ein endogenes Alloxazin ist.

3. Verfahren nach Anspruch 2, wobei der Photosensibilisator 7,8-Dimethyl-10-ribitylisoalloxazin ist.

4. Verfahren nach Anspruch 1, wobei die Photostrahlung eine oder mehr Wellenlängen im sichtbaren Spektrum umfasst.

5. Verfahren nach Anspruch 1, wobei die Photostrahlung eine oder mehr Wellenlängen im Ultraviolett-Spektrum umfasst.

6. Verfahren nach Anspruch 1, wobei auf das Fluid von Schritt (a) eine Photostrahlungsquelle in einer ausgewählten Tiefe einwirkt, um Penetration der Photostrahlung durch das Fluid und Inaktivierung des Parasiten sicher zu stellen.

7. Verfahren nach Anspruch 1, wobei das Fluid menschliche Blutbestandteile umfasst.

8. Verfahren nach Anspruch 1, wobei das Fluid menschliches Vollblut ist.

9. Verfahren nach Anspruch 1, wobei das Fluid ein abgetrenntes menschliches Blutprodukt ist.

10. Verfahren nach Anspruch 1, wobei ein endogener Photosensibilisator zu einem Antikoagulanz gegeben wird und das Antikoagulanz zu dem Fluid gegeben wird.

11. Verfahren nach Anspruch 1, wobei eine vorgemischte wässrige Lösung, umfassend den Photosensibilisator, zu dem Fluid gegeben wird.

12. Verfahren nach Anspruch 3, wobei der Photosensibilisator zu dem Fluid in einer Konzentration von 300 bis 500 $\mu$M gegeben wird.

13. Verfahren nach Anspruch 1, ferner umfassend den zusätzlichen Schritt von Einstellen des Levels von Plasma in dem Fluid.

## Revendications

1. Procédé pour traiter un fluide afin d'inactiver au moins le parasite *P. falciparum* qui peut y être présent, ledit fluide contenant un ou plus d'un composant choisi parmi du sang total humain, des constituants du sang humain ou des compositions aqueuses contenant une protéine biologiquement active dérivée du sang humain, le procédé comprenant les étapes suivantes :

   (a) ajouter au fluide une quantité essentiellement non toxique à inactivation effective d'un photosensibilisant qui est une alloxazine endogène ou une alloxazine dérivée de base endogène ; et
   (b) exposer le fluide de l'étape (a) à un photorayonnement suffisant pour activer le photosensibilisant, inactivant ainsi au moins tout *P. falciparum* présent dans le fluide.

2. Procédé selon la revendication 1, dans lequel le photosensibilisant est une alloxazine endogène.

3. Procédé selon la revendication 2, dans lequel le photosensibilisant est la 7,8-diméthyl-10-ribityl-isoalloxazine.

4. Procédé selon la revendication 1, dans lequel le photorayonnement comprend une ou plus d'une longueur d'onde dans le spectre du visible.

5. Procédé selon la revendication 1, dans lequel le photorayonnement comprend une ou plus d'une longueur d'onde dans le spectre de l'ultraviolet.

6. Procédé selon la revendication 1, dans lequel ledit fluide de l'étape (a) est exposé à une source de photorayonnement à une profondeur choisie pour garantir la pénétration du photorayonnement à travers le fluide et l'inactivation du parasite.

7. Procédé selon la revendication 1, dans lequel le fluide comprend des constituants du sang humain.

8. Procédé selon la revendication 1, dans lequel le fluide est du sang total humain.

**9.** Procédé selon la revendication 1, dans lequel le fluide est un produit de sang humain séparé.

**10.** Procédé selon la revendication 1, dans lequel le photosensibilisant endogène est ajouté à un anticoagulant et ledit anticoagulant est ajouté au fluide.

**11.** Procédé selon la revendication 1, dans lequel une solution aqueuse prémélangée comprenant le photosensibilisant est ajoutée au fluide.

**12.** Procédé selon la revendication 3, dans lequel le photosensibilisant est ajouté au fluide à une concentration de 300 à 500 μM.

**13.** Procédé selon la revendication 1 comprenant en outre l'étape supplémentaire d'ajuster le niveau de plasma dans le fluide.

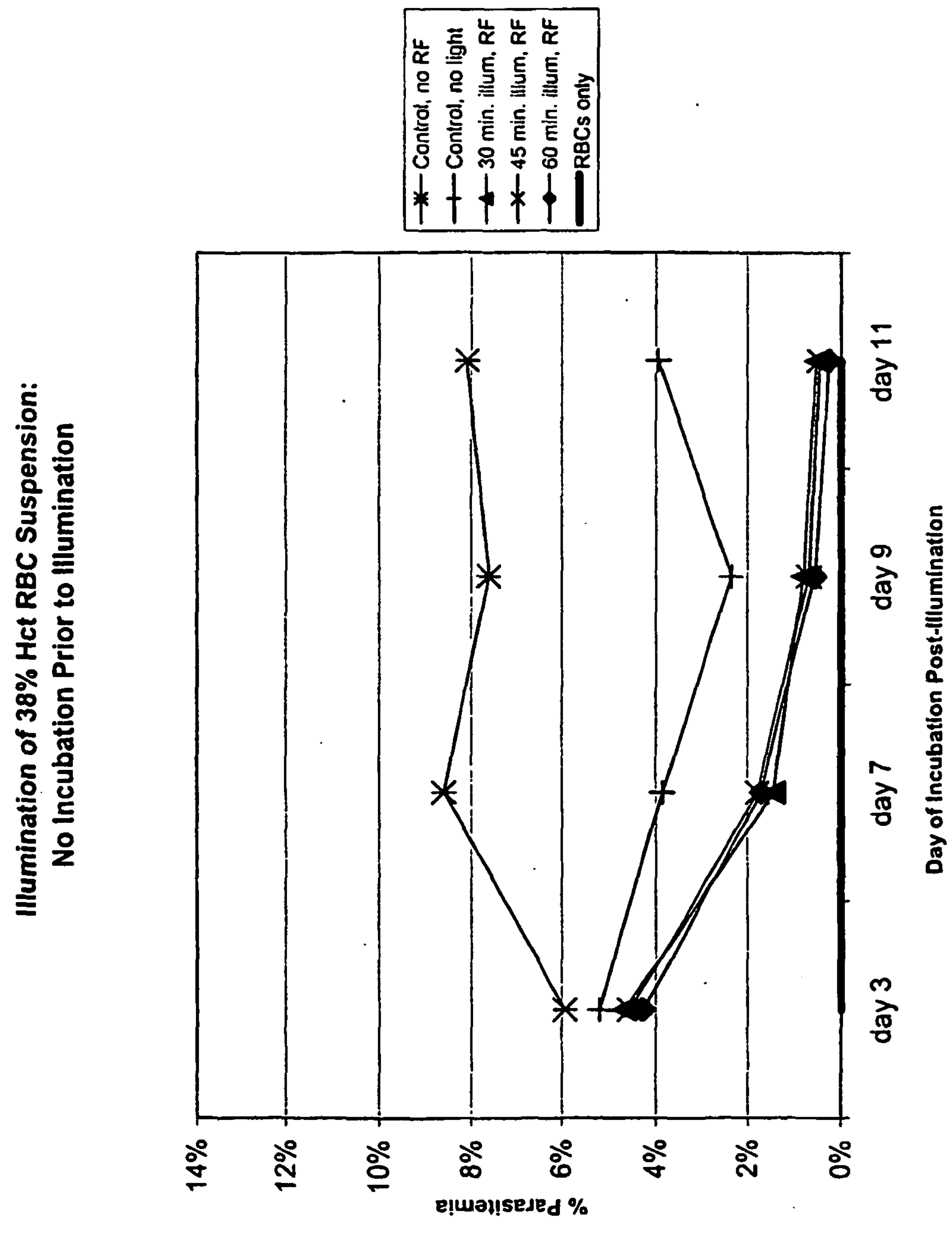
Illumination of 38% Hct RBC Suspension:
No Incubation Prior to Illumination
% Parasitemia
14%
12%
10%
8%
6%
4%
2%
0%
day 3
day 7
day 9
day 11
Day of Incubation Post-Illumination
Control, no RF
Control, no light
30 min. illum, RF
45 min. illum, RF
60 min. illum, RF
RBCs only

*Figure 1*

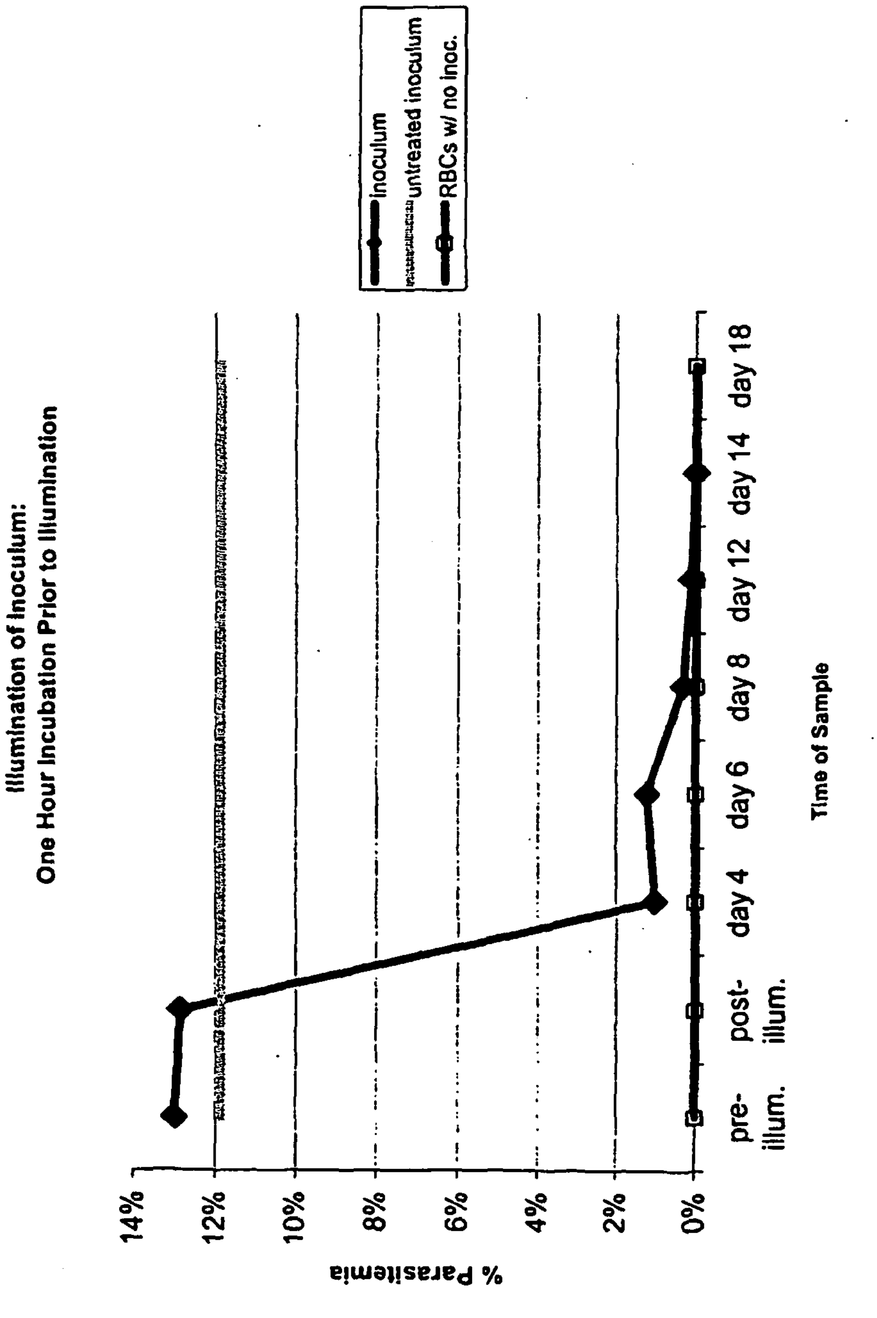

Illumination of Inoculum:
One Hour Incubation Prior to Illumination
% Parasitemia
14%
12%
10%
8%
6%
4%
2%
0%
pre-illum.
post-illum.
day 4
day 6
day 8
day 12
day 14
day 18
Time of Sample
inoculum
untreated inoculum
RBCs w/ no inoc.


*Figure 2*

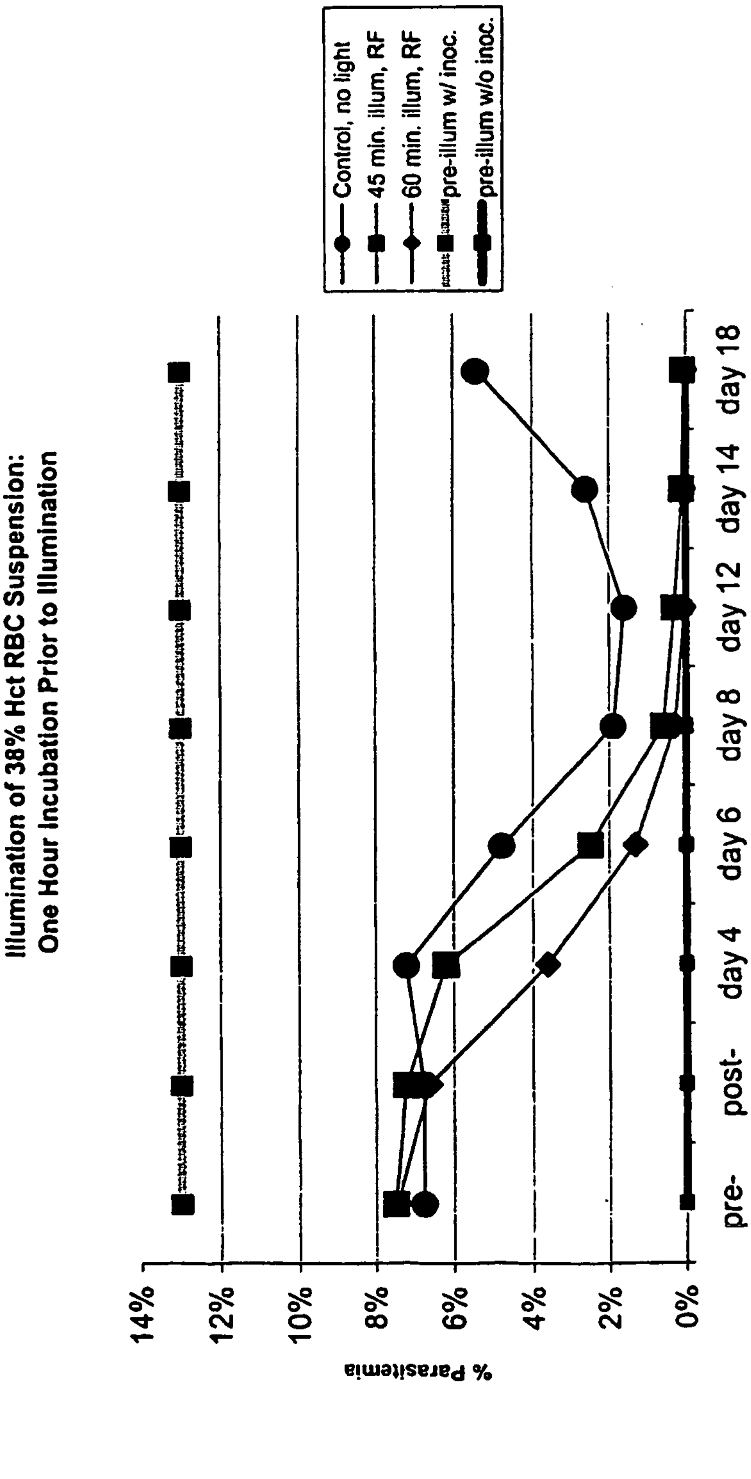
Illumination of 38% Hct RBC Suspension:
One Hour Incubation Prior to Illumination
% Parasitemia
14%
12%
10%
8%
6%
4%
2%
0%
pre-illum.
post-illum.
day 4
day 6
day 8
day 12
day 14
day 18
Time of Sample
Control, no light
45 min. illum, RF
60 min. illum, RF
pre-illum w/ inoc.
pre-illum w/o inoc.

*Figure 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 196515 A
- US 5607924 A
- US 5714328 A, Magda
- US 5041078 A, Matthews
- US 5545516 A, Wagner
- US 4915683 A
- US 5304113 A, Sieber
- US 4727027 A, Wiesehahn, G.P
- US 5516629 A, Park
- US 5587490 A, Goodrich Jr., R.P
- US 5418130 A, Platz
- US 5654443 A, Wollowitz
- US 5709991 A, Lin
- US 5120649 A
- US 5232844 A, Horowitz
- US 5360734 A, Chapman
- US 5342752 A, Platz
- US 5798238 A, Goodrich, Jr.
- US 4612007 A
- US 4683889 A, Edelson
- US 5290221 A, Wolfe, Jr.
- US 5536238 A, Bischof
- US 5527704 A, Wolf, Jr.
- US 5868695 A, Wolf, Jr.
- WO 9606647 A
- US 6258577 B
- US 6277337 B
- US 6268120 B
- WO 0128599 A
- WO 0004930 A
- US 725426 A
- US 596429 A
- WO 0196340 A
- WO 0178792 A
- WO 9428120 A
- US 777727 A
- US 5653887 A
- US 4880788 A
- US 08924519 B
- WO 9911305 A
- US 10104766 B
- US 10247262 B
- US 60368778 B
- US 10159781 B
- US 09982298 B
- US 10328717 B
- US 10065073 B
- US 09962029 B
- US 60353223 B
- US 60355393 B
- US 60377697 B
- US 10325402 B
- US 60353319 B
- US 60379328 B
- US 60375734 B
- US 60373198 B
- US 60373936 B
- US 60378374 B
- US 60375849 B
- US 09586147 B
- US 09596429 B
- US 60375670 B
- US 0221925 W
- US 60319488 B
- US 60319641 B
- US 09119666 B
- US 09357188 B
- US 09420652 B
- US 09777727 B
- US 10256852 B
- US 09725426 B


### Non-patent literature cited in the description


- **MIMS, CA et al.** *Medical Microbiology,* 1993, 30.8-30.9
- **BOYD RF.** Clinical parasitology. In Basic Medical Microbiology. Little, Brown and Company, 1995, 513-514
- Shulman I. Transmission of parasitic infections by blood transfusion. **WILLIAMS ; WILKINS.** Principles of transfusion medicine. 1996, 733-8
- **NAHLEN BL et al.** Reassessment of blood donor selection criteria for United States travelers to malarious areas. *Transfusion,* 1991, vol. 31, 798-804
- **GOODRICH, R.P. et al.** The Design and Development of Selective, Photoactivated Drugs for Sterilization of Blood Products. *Drugs of the Future,* 1997, vol. 22, 159-171

- **TSUGITA, A et al.** Photosensitized inactivation of ribonucleic acids in the presence of riboflavin. *Biochimica et Biophysica Acta,* 1965, vol. 103, 360-363
- **SPECK, W.T. et al.** Further Observations on the Photooxidation of DNA in the Presence of Riboflavin. *Biochimica et Biophysica Acta,* 1976, vol. 435, 39-44
- **KURATOMI, K. et al.** Studies on the Interactions between DNA and Flavins. *Biochimica et Biophysica Acta,* 1977, vol. 476, 207-217
- **HOFFMANN, M.E. et al.** DNA Strand Breaks in Mammalian Cells Exposed to Light in the Presence of Riboflavin and Tryptophan. *Photochemistry and Photobiology,* 1979, vol. 29, 299-303
- **PIETTE, J. et al.** Production of Breaks in Single- and Double-Stranded Forms of Bacteriophage ΦX174 DNA by Proflavine and Light Treatment. *Photochemistry and Photobiology,* 1979, vol. 30, 369-378
- **PIETTE, J. et al.** Alteration of Guanine Residues during Proflavine Mediated Photosensitization of DNA. *Photochemistry and Photobiology,* 1981, vol. 33, 325-333
- **J. CADET et al.** Mechanisms and Products of Photosensitized Degradation of Nucleic Acids and Related Model Compounds. *Israel J. Chem.,* 1983, vol. 23, 420-429
- **KORYCKA-DAHL, M. et al.** Photodegradation of DNA with Fluorescent Light in the Presence of Riboflavin, and Photoprotection by Flavin Triplet-State Quenchers. *Biochimica et Biophysica Acta,* 1980, vol. 610, 229-234
- **PEAK, J.G. et al.** DNA Breakage Caused by 334-nm Ultraviolet Light is Enhanced by Naturally Occurring Nucleic Acid Components and Nucleotide Coenzymes. *Photochemistry and Photobiology,* 1984, vol. 39, 713-716
- **AKOMPONG, T. et al.** In Vitro Activity of Riboflavin against the Human Malaria Parasite Plasmodium falciparum.,. *Antimicrob Agents Chemother,* January 2000, vol. 44, 88-96
- **AKOMPONG, T. et al.** Gametocytocidal Activity and Synergistic Interactions of Riboflavin with Standard Antimalarial Drugs against Growth of Plasmodium falciparum In Vitro. *Antimicrob Agents Chemother,* November 2000, vol. 44, 3107-3111
- **DAS BS et al.** Riboflavin deficiency and severity of malaria. *Eur J Clin Nutr,* April 1988, vol. 42, 277-83
- **DUTTA P.** Enhanced uptake and metabolism of riboflavin in erythrocytes infected with Plasmodium falciparum. *J Protozool,* September 1991, vol. 38, 479-83
- **G. APPLEYARD.** *J. Gen. Virol.,* 1967, vol. 1, 143-152
- **R SCHUYLER.** *Transfusion and Apheresis Science,* 2001, vol. 25, 189-190
- **CADET et al.** *J. Chem.,* 1983, vol. 23, 420-429
- **PONNUDURAI T et al.** The production of mature gametocytes of Plasmodium falciparum in continuous culture of different isolates infective to mosquitoes. *Trans Roy Soc Trop Med Hyg.,* vol. 76, 242
- **TRAGER W ; JENSEN JB.** Human malaria parasites in continuous culture. *Science,* 1976, vol. 193, 673
- **DRUILHE, P. et al.** A colorimetric in vitro drug-sensitivity assay for P. falciparum based on a highly sensitive two-site LDH antigen capture ELISA assay. *Am J Trop Med Hyg,* May 2001, vol. 64, 233-41